# EUROPEAN PATENT SPECIFICATION

(11) **EP 3 373 813 B1**
(45) Date of publication and mention of the grant of the patent: **30.12.2020**
(21) Application number: 16864829.3
(22) Date of filing: 08.11.2016
(51) Int. Cl.: A61B 5/15, B01D 61/38, G01N 33/48, G01N 33/49, B01D 15/10, B01D 15/26, B01L 3/00

(54) **PLASMA EXTRACTION DEVICE**
PLASMAEXTRAKTIONSVORRICHTUNG
DISPOSITIF D'EXTRACTION DE PLASMA

(30) Priority: 10.11.2015 US 201562253577 P; 07.11.2016 US 201615345079
(43) Date of publication of application: 19.09.2018
(73) Proprietor: Neoteryx, LLC, Torrance, CA 90501 (US)
(72) Inventor: KUSHON, Stuart, A., Carson, CA 90745 (US); ZAMBA, Gene, Carson, CA 90745 (US); RUDGE, James, Byron, Carson, CA 90745 (GB); JARING, Carolyn, Carson, CA 90745 (US)
(74) Representative: Boult Wade Tennant LLP
(86) International application number: PCT/US2016/060903
(87) International publication number: WO 2017/083252

(56) References cited:
- EP-A1- 1 712 180
- EP-A2- 2 375 249
- US-A- 3 937 213
- US-A- 4 228 798
- US-A- 6 139 757
- US-A1- 2006 180 542
- US-A1- 2010 324 449
- US-A1- 2012 275 955

## Description

### BACKGROUND OF THE DISCLOSURE

### Field of the Disclosure

One or more examples of the present disclosure relate to a sample collection device.

### Description of Related Art

Conventional methods of extraction or separation of plasma are well known and have been in use for a number of years, which include centrifugation, pressure induced plasma separation devices, volume induced plasma separation device, etc.

Centrifugation is a very well known method used for separating plasma, which requires the use of complex devices and further, complex methods and systems for sample tracking (allocating, labeling, etc.) the extracted liquid plasma for safe transport and continuous association with a test subject. Once separated by centrifugation, the actual extraction of liquid plasma itself is a non-automated process, requiring the use of skilled lab technicians that may inadvertently introduce operator errors in the extraction process of the liquid plasma and also add to the overall cost.
Centrifugation has a major disadvantage in that it cannot be easily used to generate plasma at the point of care.

Pressure (positive or negative - vacuum) induced plasma generation may use conventional pumps (very large and complex) to force liquid (e.g., blood) through a well-known plasma separator to generate liquid plasma. A non-limiting example of a plasma separator is VIVID PLASMA SEPARATOR MEMBRANE™ manufactured by PALL CORPORATION. Drawbacks with currently available pressure induced plasma generation systems are similar to centrifugation systems with respect to the use of additional equipment, need for complex sample tracking, use of skilled lab technicians, and accounting for operator errors. It should be noted that convention pressure induced plasma generation (positive or negative - vacuum) move wet "plasma" fluid into a tube for later analysis, which is an additional drawback and may be considered as bio-hazard in certain jurisdictions.

A volume induced plasma generation may also use the well-known plasma separator with a conventional lateral flow device. In volume induced plasma generation schemes, fairly large volume of liquid (for example, large volume of water mixed with desired amount of blood) is poured onto a container that holds the plasma separator, with blood plasma generated due to sheer volume of liquid continuously passing through the plasma separator. The lateral flow device may then absorb the generated plasma by capillary action. It should be noted that an additional drawback with volume induced plasma generation is dilution of plasma and hence, loss in quantitative knowledge of plasma concentration resulting in qualitative rather than quantitative assay.
EP 2375249 relates to a device for separating plasma from a blood sample comprising a stacked structure which is provided with a first portion including a separating member having a first surface for applying or receiving the blood sample, wherein the separating member is adapted to permit the passage of plasma but to inhibit the passage of cells, and a second portion including an absorptive member for absorbing the plasma, which has a second surface in contact with the separating member for receiving the plasma, wherein the absorptive member is adapted to generate a capillary pressure so as to draw plasma from the separating member to the absorptive member. US 2010/0324449 relates to a device for taking blood by capillarity and incorporating a filter and downstream therefrom, an absorbent material for separating a plasma phase of the blood from a cell phase.

Accordingly, in light of the current state of the art and the drawbacks to current plasma extraction methods mentioned above, a need exists for plasma extraction system and method that would use capillary action (or gravity) as a motive force to extract accurate quantity (amount) of plasma and hence, known concentration of plasma from a source of liquid without the use of external devices such as centrifuges, pumps, additional volume of liquid, etc. Further, a need exists for plasma extraction system and method that would use capillary action (or gravity) as a motive force to extract accurate quantity (amount) of plasma and hence, known concentration of plasma from a source of liquid without the use of external devices such as centrifuges, pumps, additional volume of liquid, etc. Further, a need exists for plasma extraction system and method that would enable dry transport of fixed, predetermined quantity of plasma, even if the generated plasma is pressure (positive or negative - vacuum) induced.

### BRIEF SUMMARY OF THE INVENTION

In accordance with the present invention, there is provided
a device for extraction of plasma from a liquid sample, comprising:
a first absorbent member that wicks the liquid sample;
a second absorbent member that retains a fixed, predetermined quantity of plasma; and
a separator placed in physical contact between the first absorbent member and the second absorbent member for generating plasma from the liquid sample;
a first side of the separator is comprised of a first membrane and a second side of the separator is comprised of a second membrane;
the first membrane is comprised of a lower porosity construction with enhanced hydrophilic properties relative to the first absorbent member, facilitating capillary action from the first absorbent member to the first membrane;
the second membrane is comprised of a higher porosity construction and lesser hydrophilic properties relative to the second absorbent member, facilitating capillary action from the second membrane to the second absorbent member;
wherein: the plasma loaded second absorbent member is dry-transferred for assay

In addition, further advantageous embodiments follow from the dependent claims.

### SUMMARY OF THE DISCLOSURE

Another non-limiting, exemplary aspect of an example of the present disclosure provides a method for extraction of plasma, comprising:
wicking a volume of a liquid sample from a liquid source through a first capillary action;
wicking the liquid sample to a separator through a second capillary action, with the separator generating a volume of a plasma;
wicking a fixed, predetermined quantity of the plasma from the separator through a third capillary action;
storing and dry-transferring of the collected plasma for assay.

The second capillary action is mostly driven by differential porosity construction, and the third capillary action is mostly driven by differential in hydrophilic properties.

Yet another non-limiting, exemplary aspect of an example of the present disclosure provides a device, comprising:
a handler assembly; and
a plasma extractor module;
wherein: the plasma extractor module is detachably associated with the handler assembly.

A further non-limiting, exemplary aspect of an example of the present disclosure provides a device, comprising:
a handler assembly comprised of:
a handler that houses an absorbent reservoir of a plasma extractor assembly; and
a plasma extractor module that is detachably friction-fit secured to the handler assembly and includes a separator and an absorbent probe of the plasma extractor assembly.

Yet a further non-limiting, exemplary aspect of an example of the present disclosure provides a device for extraction of plasma from a liquid sample, comprising:
a housing having a first piece and a second piece;
the first piece includes one or more openings to frictionally secure one or more absorbent probes, with the second piece having at least one opening to frictionally secure at least one absorbent reservoir;
the first piece and the second piece forming a compartment when assembled within which a separator is housed in physical contact in between the absorbent probe and the absorbent reservoir.

Another non-limiting, exemplary aspect of an example of the present disclosure provides a container, comprising:
a tube configured assembly with air evacuated from within to create negative air pressure inside the tube assembly;
the tube assembly includes:
   a first detachable closure to air-tight close a first open end of the tube assembly; and
   a second detachable closure to air-tight close a second open end of the tube assembly.

Yet another non-limiting, exemplary aspect of an example of the present disclosure provides a device for extraction of plasma from a liquid sample, comprising:
a tube assembly with a detachable first closure and a detachable second closure with air evacuated from within to generate absolute lower air pressure inside the tube;
the air evacuated tube includes:
   a first opening that is airtight closed by the first detachable closure;
   a second opening that is airtight closed by the detachable second closure; and
   a plasma extraction device that is housed inside the air-evacuated tube assembly, and removable through one of first and second opening.

A further non-limiting, exemplary aspect of an example of the present disclosure provides a device for extraction of plasma from a liquid sample, comprising:
a hermetically sealed air-evacuated tube assembly to draw liquid sample inside the tube assembly driven by pressure differential between inside and outside the tube assembly;
a first absorbent member that wicks fixed, predetermined quantity of liquid sample;
a second absorbent member that retains plasma; and
a separator placed in physical contact between the first absorbent member and the second absorbent member for generating plasma from liquid sample;
wherein: the plasma loaded second absorbent member is removed from tube assembly and dry-transferred for assay.

Yet a further non-limiting, exemplary aspect of an example of the present disclosure provides a device for extraction of plasma from a liquid sample, comprising:
a tube assembly for drawing liquid sample inside the tube assembly driven by pressure differential between inside and outside the tube assembly generated by a pressure differential generator;
a plasma extraction device positioned inside the tube assembly, comprising:
   a first absorbent member that wicks fixed, predetermined quantity of liquid sample drawn into the tube assembly;
   a second absorbent member that retains fixed, predetermined quantity of plasma; and
   a separator placed in physical contact between the first absorbent member and the second absorbent member for generating plasma from liquid sample;
   wherein: the plasma loaded second absorbent member is removed from tube assembly and dry-transferred for assay.

Another non-limiting, exemplary aspect of an example of the present disclosure provides a device for extraction of plasma from a liquid sample, comprising:
pressure differential generator for moving liquid sample from a source via an invasive probe and into a collection chamber of an intermediate adapter connected to the pressure differential generator; and
a plasma extractor module positioned within the intermediate adapter, with an absorbent probe of the plasma extractor module extended to within the collection chamber, near egress opening of the invasive probe for receiving liquid sample.

Yet another non-limiting, exemplary aspect of an example of the present disclosure provides a device for extraction of plasma from a liquid sample, comprising:
a plasma extraction device positioned within a tube assembly;
the tube assembly is comprised of:
   a top closure; and
   a lateral pressure differential generation outlet adapted to be detachably associated with a pressure differential generator.

These and other features and aspects of the invention will be apparent to those skilled in the art from the following detailed description of preferred non-limiting exemplary embodiments, taken together with the drawings and the claims that follow.

### Brief description of the drawings

It is to be understood that the drawings are to be used for the purposes of exemplary illustration only and not as a definition of the limits of the invention. Throughout the disclosure, the word "exemplary" may be used to mean "serving as an example, instance, or illustration," but the absence of the term "exemplary" does not denote a limiting embodiment. Any embodiment described as "exemplary" is not necessarily to be construed as preferred or advantageous over other embodiments. In the drawings, like reference character(s) present corresponding part(s) throughout

It is noted that references to "embodiments" throughout the description which are not under the scope of the appended claims represent possible examples and are therefore not part of the present invention unless the context clearly dictates otherwise. The invention is defined by the appended claims.
FIGS. 1A to 1N are non-limiting, exemplary illustrations of various views of a plasma extraction device for extraction of plasma from a liquid sample in accordance with one or more embodiments of the present invention;
FIGS. 2A to 2R are non-limiting, exemplary illustration of various views of a plasma extraction device for extraction of plasma from a liquid sample in accordance with one or more embodiments of the present invention;
FIGS. 3A to 3F are non-limiting, exemplary illustration of various views of a plasma extraction device for extraction of plasma for a liquid sample in accordance with one or more embodiments of the present invention;
FIGS. 4A to 4C are non-limiting, exemplary illustration of various views of a plasma extraction device for extraction of plasma from a liquid sample in accordance with one or more embodiments of the present invention;
FIGS. 5A to 5H are non-limiting, exemplary illustration of various views of a plasma extraction assembly for extraction of plasma from a liquid sample in accordance with one or more embodiments of the present invention;
FIGS. 6A to 6F are non-limiting, exemplary illustration of various views of a plasma extraction assembly for extraction of plasma from a liquid sample in accordance with one or more embodiments of the present invention;
FIGS. 7A to 7P are non-limiting, exemplary illustration of various views of a plasma extraction assembly for extraction of plasma from a liquid sample in accordance with one or more embodiments of the present invention; and
FIGS. 8A-1 to 8D are non-limiting, exemplary illustration of various views of a plasma extraction assembly for extraction of plasma from a liquid sample in accordance with one or more embodiments of the present invention.

### DETAILED DESCRIPTION OF THE INVENTION

The detailed description set forth below in connection with the appended drawings is intended as a description of presently preferred embodiments of the invention and is not intended to represent the only forms in which the present invention may be constructed and or utilized.

It is to be appreciated that certain features of the invention, which are, for clarity, described in the context of separate embodiments, may also be provided in combination in a single embodiment. Conversely, various features of the invention that are, for brevity, described in the context of a single embodiment may also be provided separately or in any suitable sub-combination or as suitable in any other described embodiment of the invention. Stated otherwise, although the invention is described below in terms of various exemplary embodiments and implementations, it should be understood that the various features and aspects described in one or more of the individual embodiments are not limited in their applicability to the particular embodiment with which they are described, but instead can be applied, alone or in various combinations, to one or more of the other embodiments of the invention.

Throughout the disclosure, the term "separator" refers to filter membranes, non-limiting, non-exhaustive listing of examples of which may include nylon filters, cellulous filters, polyethylene filters, etc. Very specific, non-limiting examples of filter membranes (i.e., separators) that may be used in accordance with one or more embodiments of the present invention for example, are various types of VIVID PLASMA SEPARATOR MEMBRANE™ manufactured by PALL CORPORATION.

In general, a separator used in accordance with one or more embodiments of the present invention may be composed of material that may filter fluid based on non-limiting, exemplary factors such as size, filter porosity (e.g., pour diameter), filter depth, or other factors that enhance high probability capture event with improved interconnected capillary system for superior capillary action without blockage. It should be noted that filter "depth" may be a function of networked tortuous path through which fluid may be traversed and hence, does not necessarily imply "thickness."

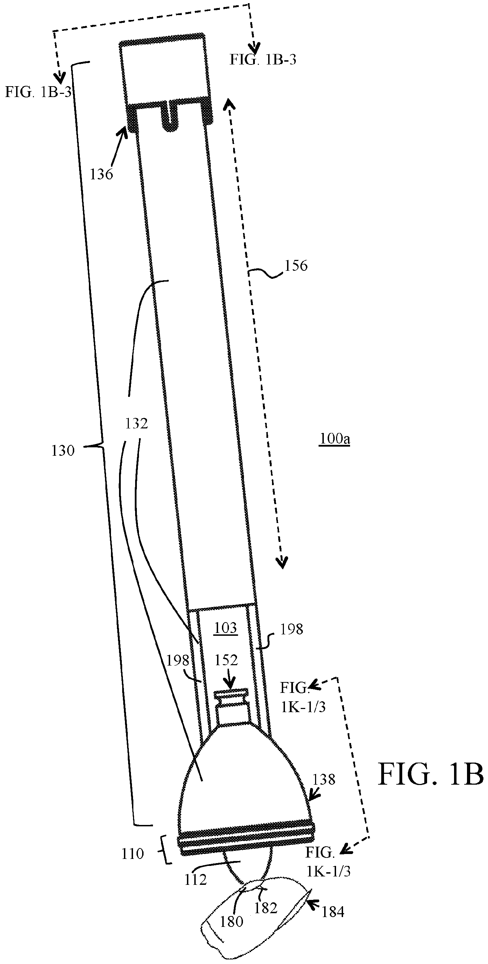

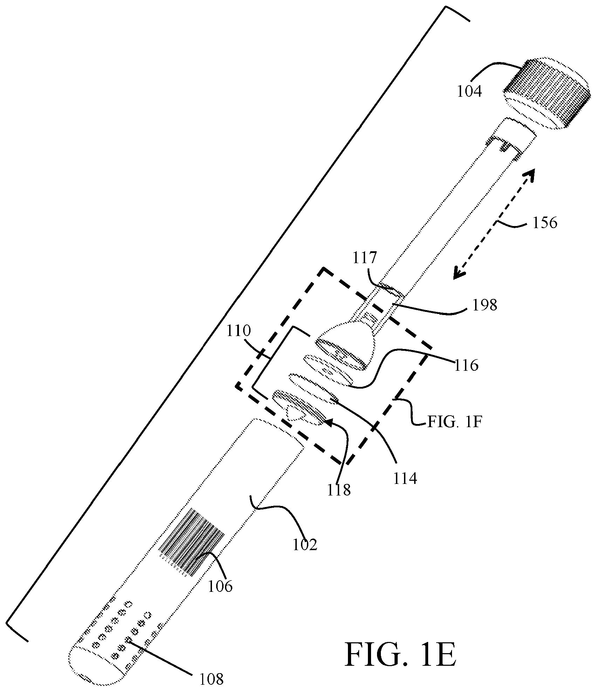

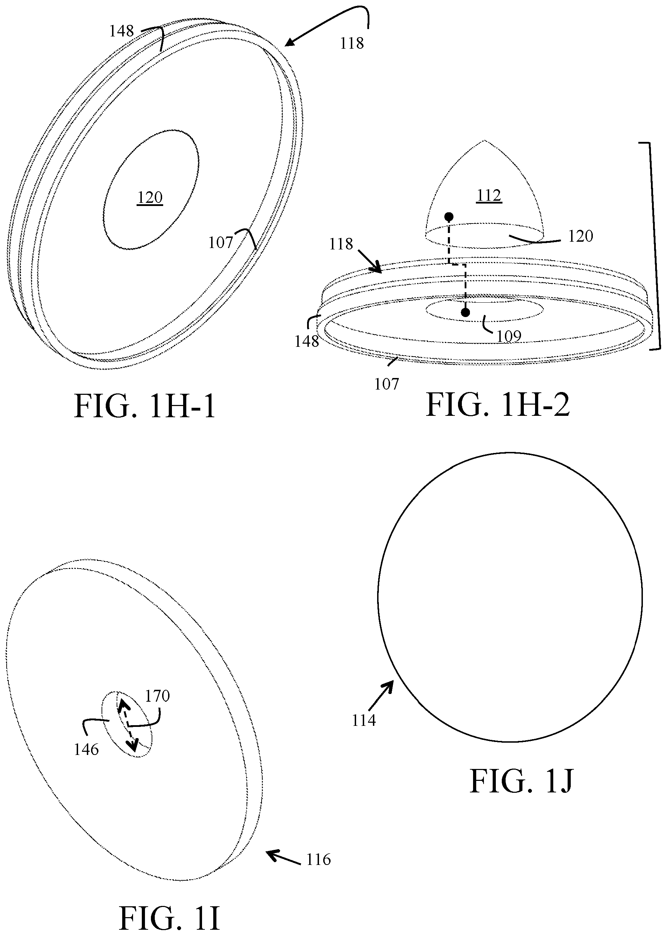

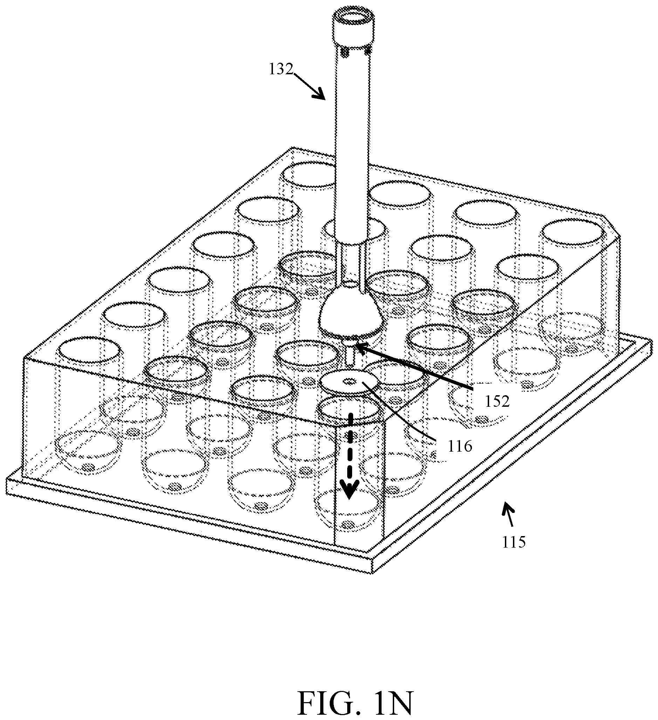

It should be noted that it is only for convenience of example and discussion purposes that throughout the disclosure liquid source 180 (FIG. 1B) is indicated from a finger prick. It will be quickly apparent that any one of the one or more embodiments disclosed may use liquid source 180 to generate plasma that is not from a finger prick.

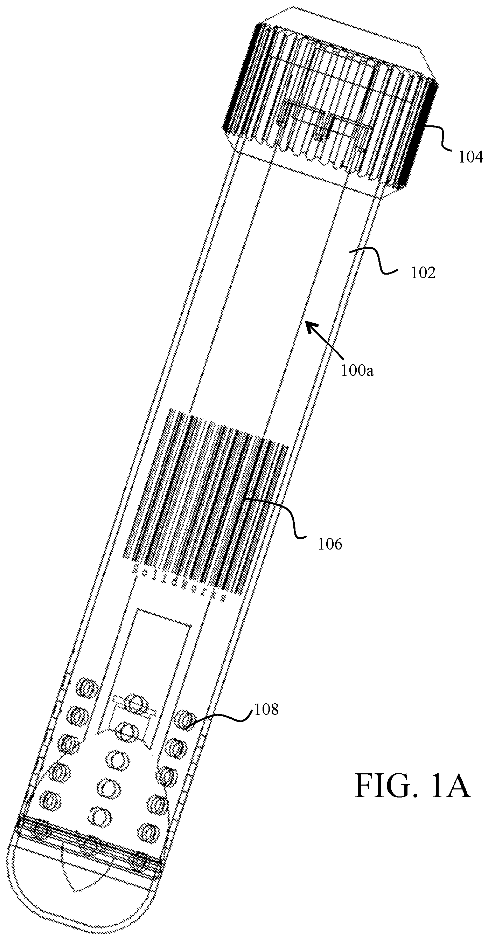

FIGS. 1A to IN are non-limiting, exemplary illustrations of various views of a plasma extraction device for extraction of plasma from a liquid sample in accordance with one or more embodiments of the present invention. FIG. 1A is a non-limiting, exemplary illustration of a plasma extraction device 100a inside a tube enclosure 102 in accordance with one or more embodiments of the present invention. As illustrated in FIG. 1A, plasma extraction device 100a with loaded plasma may be securely stored and dry-transported within tube 102 with a removable cap 104, with tube 102 including marking (such as a bar code or QR™ code) 106 for tracking purposes.

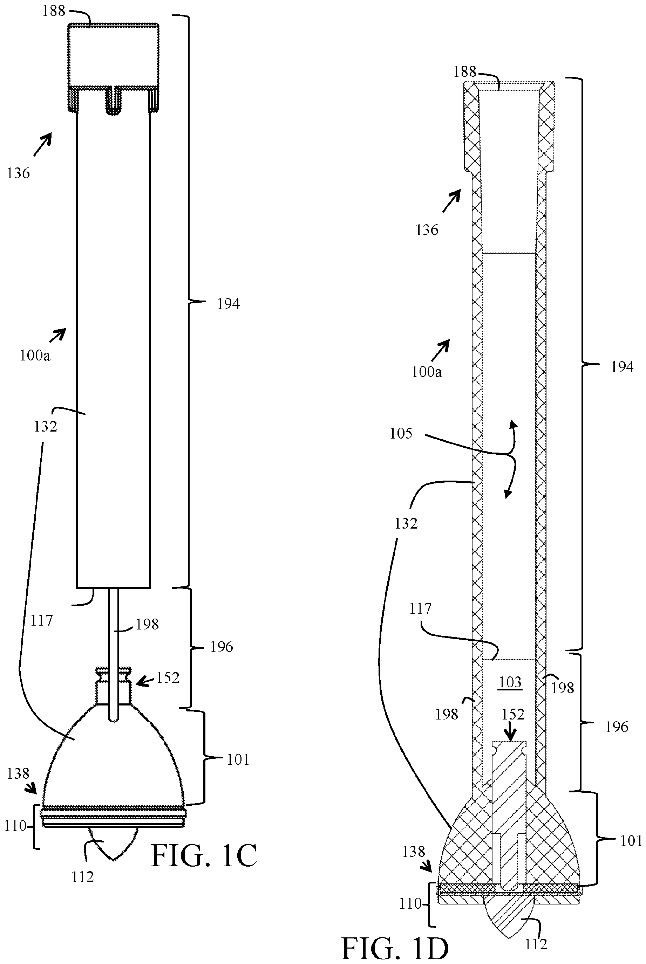

FIGS. 1B and 1C are non-limiting, exemplary illustrations of front and side views of plasma extraction device 100a, with FIG. 1D a sectional view of FIG. 1B in accordance with one or more embodiments of the present invention. As best illustrated in FIGS. 1B and 1C, users may remove plasma extraction device 100a out of tube 102, dip its absorbent probe 112 into a liquid source 180 (for example, from cut 182 of finger 184) to extract plasma from liquid sample, and place back plasma extraction device 100a securely within tube 102, enabling the liquid sample plasma to dry via vent holes 108 on tube 102 (shown in FIG. 1A). It should be noted that tube 102 and cap 104 are adapted to be operated by well known automated instruments for plasma analysis and hence, need not be handled or operated by individuals.

FIG. IE is a non-limiting exemplary exploded view illustration of the various components of plasma extraction device 100a and tube 102 in accordance with one or more embodiments of the present invention. The exploded view shown in FIG. IE illustrates disassembled, separated components that show the cooperative working relationship, orientation, positioning, and exemplary manner of assembly of the various components of plasma extraction device 100a and tube 102 in accordance with one or more embodiments of the present invention, with each component detailed below.

Referring back to FIGS. 1B and 1C, plasma extraction device 100a is comprised of a handler assembly 130 and a plasma extractor module 110 that is detachably associated with handler assembly 130. Handler assembly 130 is comprised of a handler 132 that is adapted to be used with well known automated liquid handling instruments, and includes a dislodgement mechanism 152 for dismounting of plasma extractor module 110.

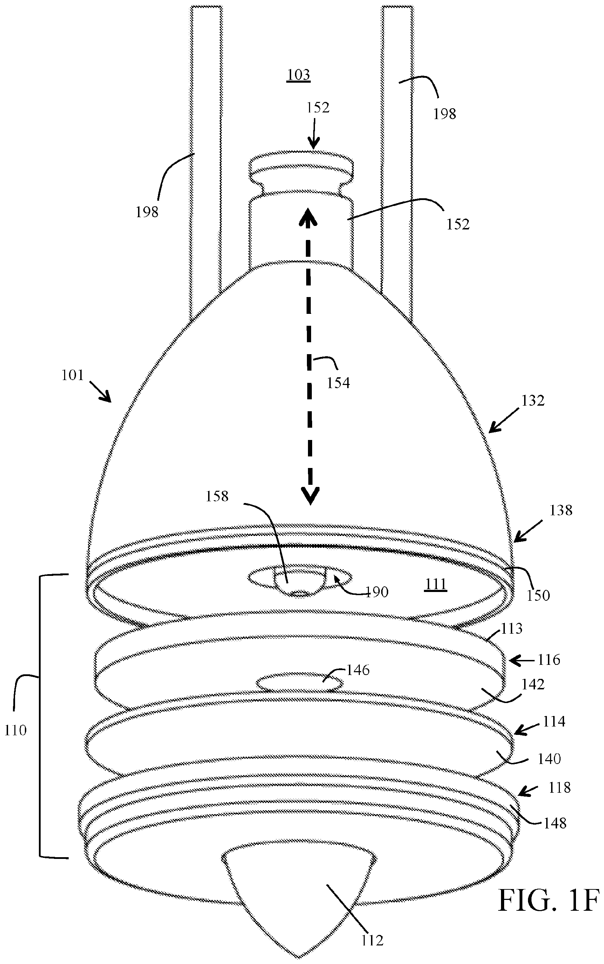

As best illustrated in FIG. 1C and sectional view FIG. 1D, in this non-limiting, exemplary instance, handler 132 is a single piece unit. A first section 194 (including first distal end 136) of handler 132 of handler assembly 130 may vary in design, is well known, and is disclosed in U.S. Patent Application publication US 2013/0116597 to Rudge et al. and U.S. Provisional Patent Application 62/149,415 to Emmet Welch, U.S. Non-Provisional Patent Application 15/130,373 to Emmet Welch. First section 194 is cylindrical and hollow 105 with top opening 188 and bottom opening 117. Second section 196 of handler 132 includes extension posts 198 that accommodate dislodgment mechanism 152 in between space 103 and connect first section 194 to a third section 101. Second distal end 138 of handler 132 (at third section 101) is configured to detachably receive and hold the detachable plasma extractor module 110, and includes a generally, flat bottom end 111 (best shown in FIG. 1F). It should be noted that handler 132 of handler assembly 130 may be easily re-configured and adapted to operate with existing automated plasma instruments without departing from the scope of the disclosure and hence, the configuration of handler 132 of handler assembly 130 illustrated should not be limiting.

As best illustrated in FIGS. 1F to 1J, plasma extractor module 110 is comprised of a housing 118 that includes a plasma extractor assembly. Plasma extractor assembly includes first absorbent member 112 (as the "probe") and hence, referred to as "absorbent probe 112," and a second absorbent member 116 (as the "reservoir" that holds the plasma) and hence, referred to as "absorbent reservoir 116." It should be noted that first and second absorbent members 112 and 116 may be identical in all aspects, including form-factor. Alternatively and as illustrated, they may also be different in form or, comprised of different materials, etc. Non-limiting, non-exhaustive listing of examples of materials for absorbent member may comprise of pores plastic, ceramic, carbon, etc. so long as the absorbent members are highly hydrophilic or chemically changed to become hydrophilic. Non-limiting, non-exhaustive listing of examples of absorbent members that may be used within one or more embodiments of the present disclosure as absorbent probe/reservoir may include those that are disclosed in U.S. Patent Application Publication 2013/0116597 to Rudge et al., U.S. Provisional Patent Application 62/149,415 to Emmet Welch, U.S. Non-Provisional Patent Application 15/130,373 to Emmet Welch, and U.S. Provisional Patent Application 62/143,696 to Gijbertus G. Rietveld, U.S. Non-Provisional Patent Application 15/048,859 to Gijbertus G. Rietveld. As further illustrated in FIGS. 1F to 1J, the plasma extractor assembly further includes a well known separator 114 (e.g., VIVID PLASMA SEPARATION MEMBRANE from PALL CORPORATION) positioned in between absorbent probe 112 and absorbent reservoir 116.

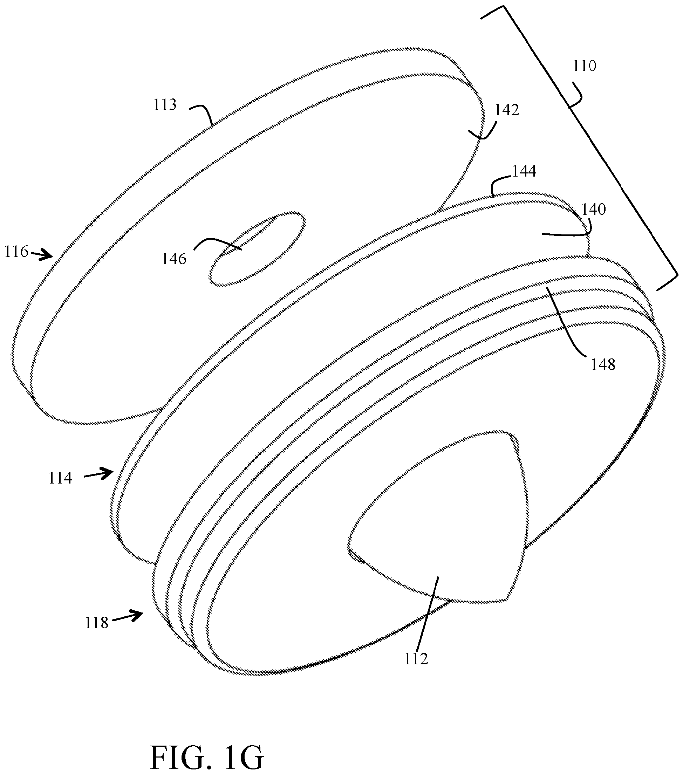

Absorbent probe 112 is physically mounted onto housing 118, with a first side 120 of absorbent probe 112 physically pressed against and contacting a first side 140 (FIGS. 1F and 1G) of separator 114. As best illustrated in FIGS. 1F, 1H-1 and 1H-2 housing 118 includes a periphery 148 with internal annular protuberance or flange 107 that is adapted to detachably couple with (e.g., detachably "snap" or press-fit within) receiving recess 150 of second distal end 138 of handler 132 to thereby detachably secure the plasma extractor assembly as the illustrated plasma extractor module 110 with handler assembly 130. Absorbent probe 112 is simply friction (or press) fit within opening 109 of housing 118, as shown in FIG. 1H-2.

Referring to FIGS. 1F and 1G, absorbent reservoir 116 has a first side 142 pressed against second side 144 of separator 114. In general, absorbent reservoir 116 is annular, with an opening 146 for operation of dislodgement mechanism 152. As indicated above, absorbent reservoir 116 may be comprised of any shape, including polygonal configurations, but optimally, it is best if absorbent reservoir 116 is configured commensurate to the shape of separator 114 for maximum contact surface area.

Both absorbent reservoir 116 and separator 114 may have complementary undulating surfaces to maximize surface-to-surface contact area without increasing the diameter of either absorbent members 112 and 116 or separator 114. In fact, aspects that would increase or maximize surface-to-surface contact area would improve efficiency and robustness (durability) of the entire system in terms of extracting the maximum amount of plasma.

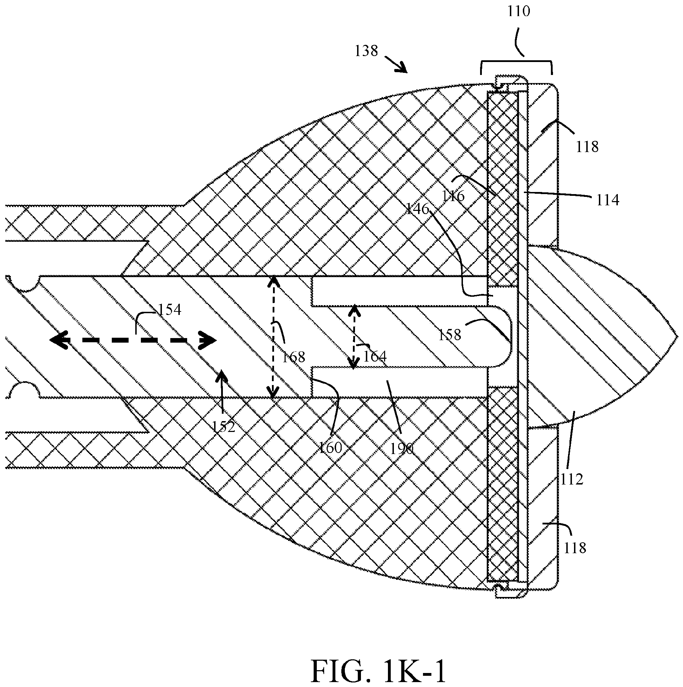

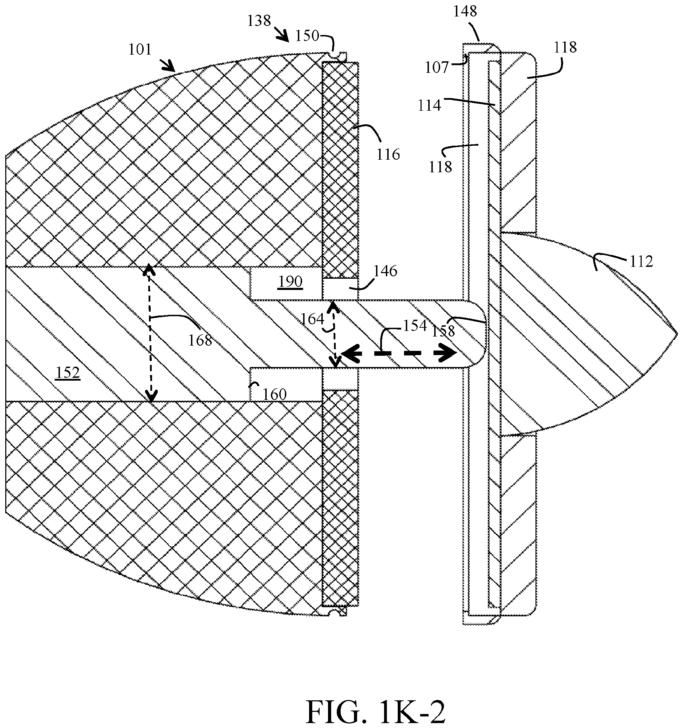

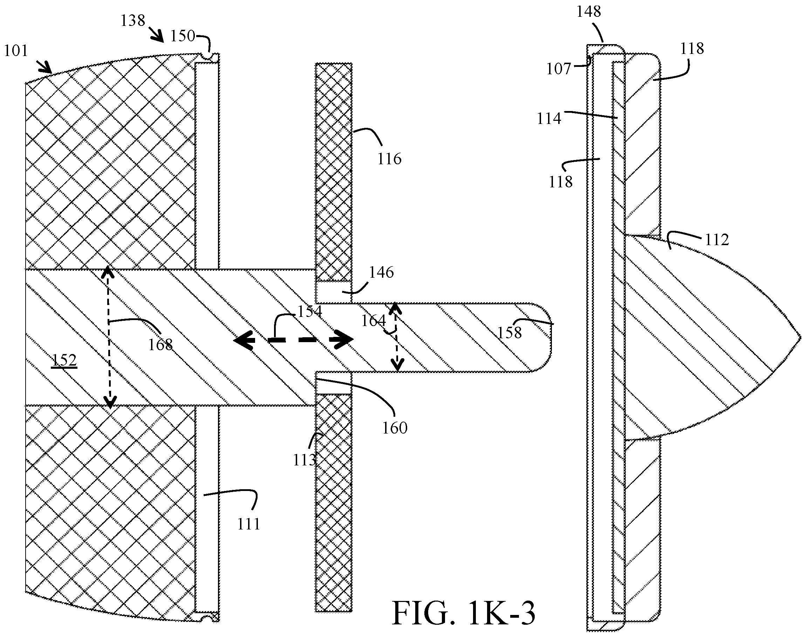

In operation, fluid sample may first be collected by absorbent probe 112 from liquid sample source 180 (FIG. 1B), and through capillary action fixed, predetermined quantity of plasma is collected and loaded onto absorbent reservoirs 116. Thereafter, plasma extraction device 100a, which now includes handler assembly 130 with plasma extractor module 110 (with plasma loaded absorbent reservoir 116) may be placed back into tube 102 (as shown in FIG. 1A) and transported dry to a lab on a well known tube tray (not shown) for automated analysis. Non-limiting examples of modes of drying may include desiccant or leaving to dry on the bench before shipping. Well known automated liquid sample handling instruments may than be used to automatically pick and uncap tube 102, and actually lift plasma extraction device 100a via top distal end 136 of handler 132 of handler assembly 130. Thereafter, plasma extractor module 110 may be dislodged from handler assembly 130 (FIGS. 1K-1 and 1K-2) by the automated liquid handling instruments but with the dry plasma loaded absorbent reservoir 116 intact and still associated with handler assembly 130. Once dislodged, the automated plasma analysis instruments then eject the dry plasma loaded absorbent reservoir 116 (FIG. 1K-2) onto well known analysis tray (FIG. IN) for analysis of the dried plasma in well-known manner.

FIGS. 1K-1 to 1K-3 are sectional views of the lower end of handler assembly 130 taken from FIG. 1B. As best illustrated in FIGS. 1K-1 to 1K-3, in this non-limiting exemplary instance, handler assembly 130 has dislodgement mechanism 152 in the form of an ejection pin (or plunger) that may be moved along a linear reciprocating path 154, parallel a longitudinal axis 156 (FIG. IE) of handler 132 of handler assembly 130 manually or by well known automated liquid sample handling instruments. Ejection pin 152 is comprised of a first engaging surface 158 for ejecting plasma extractor module 110 (but without absorbent reservoir 116), and a second engaging surface 160 for ejecting absorbent reservoir 116. In other words, as shown in FIGS. 1K-1 and 1K-2, ejection pin 152 first ejects (pushes out or away) absorbent probe 112, housing 118, and separator 114 of plasma extractor module 110, while absorbent reservoir 116 continues to remain mounted on handler assembly 130. Once handler assembly 132 and remaining absorbent reservoir 116 are brought aligned with an analysis tray 115 by a well known automated instrument (FIG. IN), ejection pin 152 is moved again (best shown in FIG. 1K-3) along linear reciprocating path 154 where second engagement surface 160 contacts and pushes top surface 113 of absorbent reservoir 116 (near periphery of opening 146) to eject absorbent reservoir 116 onto known tray 115 (as best illustrated FIG. IN). It should be noted that as is well known, the automated plasma analysis instruments may handle multiple plasma loaded extraction devices and handler assemblies 132 simultaneously.

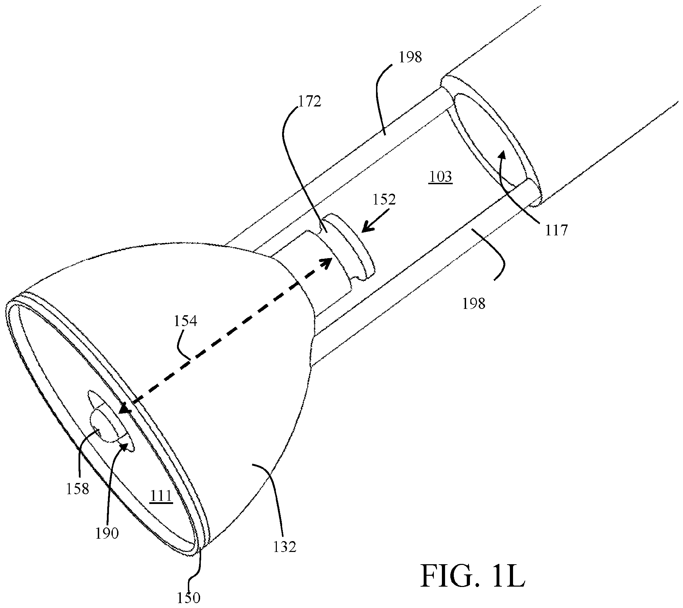

Accordingly, the automated plasma analysis instrument may move ejection pin 152 to a first position (within chamber 190 - FIGS. 1K-1 and 1K-2) to enable first engagement surface 158 to engage and dislodge absorbent probe 112, housing 118, and separator 114. As shown in FIG. 1K-3, ejection pin 152 is also moved to a second position (again by the automated liquid handling instruments) to enable second engagement surface 160 to eject absorbent reservoir 116. Ejection pin 152 and its operation may be thought of as a two-stage plunger operation, with first stage (FIGS. 1K-1 and 1K-2) releasing or dislodging absorbent probe 112, housing 118, and separator 114, and second stage (FIG. 1K-3) dislodging absorbent reservoir 116. FIG. 1L is a non-limiting, exemplary illustration of a handler assembly 130 with plasma extractor module 110 dismounted in accordance with the present invention.

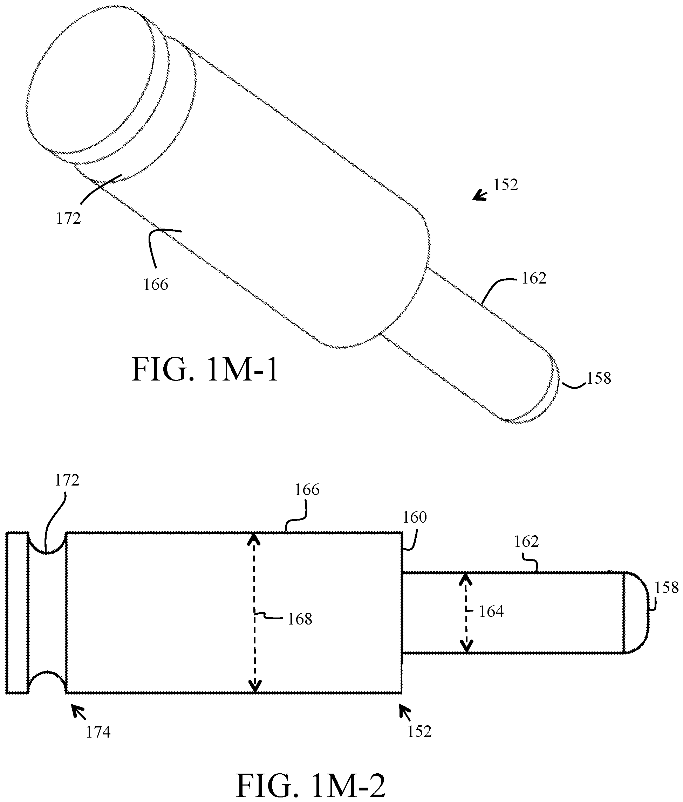

FIGS. 1M-1 and 1M-2 are non-limiting, exemplary illustrations of a dislodgement mechanism in accordance with one or more embodiments of the present invention. As illustrated, dislodgement mechanism (or ejection pin 152) is comprised of single piece unit comprised of a first section 162 with a first diameter 164 and a second section 166 with a second diameter 168 that is wider than first diameter 164. As shown, second engagement surface 160 (defined by diameter 168) has a larger expanse than a diameter 170 (FIG. 1I) of opening 146 of absorbent reservoir 116, whereas first engagement surface 158 is a smaller with smaller diameter 164 and hence, passes through opening 146 of absorbent reservoir 116. Well known automated plasma analysis instrument moves pin 152 by griping groove 172 at top distal end 174 of pin 152.

Plasma extractor module 110 enables extraction and loading of fixed, predetermined quantity of plasma from fluid sample using absorbent probe 112 that wicks liquid sample by capillary action. Fluid sample may first be collected by absorbent probe 112 contacting fluid sample source 180 and through capillary action plasma is eventually collected and loaded onto absorbent reservoirs 116. Since the size of absorbent probe 112 is known, the accurate amount of fluid sample collected by absorbent probe 112 from fluid source 180 is known. As a non-limiting example, absorbent probe 112 may have a fairly large volume size of about 10 to 500 µL or so, and may have a large porosity construction (channels) of about 40 microns.

Absorbent probe 112 has side 120 pressed against first side 140 of separator 114, which enables transfer of liquid sample by capillary action from absorbent probe 112 to first side 140 of separator 114. Separator 114 separates plasma of the transferred liquid sample in well known methods, moved from first side 140 of separator 114 to second side 144 of separator 114 (generally by capillary action).

Absorbent reservoir 116 has a first side 142 pressed against second side 144 of separator 114 to wick the plasma from second side 144 of separator 114 by capillary action. In this non-limiting, exemplary instance shown in FIGS. 1A to IN the motive force to extracting plasma from fluid sample is capillary action.

The dynamics of the capillary action between absorbent probe 112 and first side 140 of separator 114 is dominated by first side (first membrane) 140 of separator 114 due to lower porosity construction of first membrane (about 2-3 micron) compared with high porosity of absorbent probe 112 (about 40 micron). Smaller diameter structure of first side 140 of separator 114 will pull liquid from larger diameter structure of absorbent probe 112, due to the nature of capillary action. Blood cells with larger diameters 6-8 microns become trapped in first membrane (or first side 140) of separator 114, but the plasma is traversed to second membrane (or second side 144) of separator 114.

Absorbent reservoir 116 also has a large porosity construction (channels) of about 40 microns and is hydrophilic. In the final stage, it is the strong hydrophilic nature of absorbent probe 116 that dominates in the extraction of the generated plasma from separator 114. The material for the absorbent members (probe 112 and reservoir 116) is modulated chemically in well-known methodologies to have an extremely high affinity for liquid to readily wick fluid.

First side 140 of separator 114 is comprised of first membrane and second side 144 of separator 114 is comprised of a second membrane. First membrane is comprised of low porosity construction (e.g., may have channels of about 2-3 micron in diameter) and may also be optionally highly hydrophilic. The low porosity blocks particulates larger than 2-3 micron (for example, erythrocytes (red blood cells) are around 6-8 micron and leukocytes (white blood cells) are 12-17 microns). Second membrane is comprised of high porosity construction (e.g., may have channels of about 20 to 30 microns in diameter) and may also be optionally partially hydrophilic. In general, separator 114 is preferred to be larger size (e.g., in diameter) due to splaying of the fluid.

It is important to note that absorbent reservoir 116 extracts specific quantity of stored plasma from second membrane (or second side 144) of separator 114 due to differences in hydrophilic nature of absorbent reservoir 116 and second membrane of separator 114 and also the size of absorbent reservoir 116. Absorbent reservoir 116 is highly hydrophilic and also is porous (about 40 micron) whereas second membrane of separator 114 may potentially be partially hydrophilic. In other words, the motive that drives the capillary action is the hydrophilic nature of absorbent reservoir 116 in the dynamics between absorbent reservoir 116 and separator 114.

Absorbent reservoir 116 has known fixed volumetric porous volumes, which would enable it to retain or hold a known fixed volume of plasma (e.g., 5 micro-liters, or 10 micro-liters, or others such as 30 micro-liters, and so on. Once absorbent reservoir 116 is filled with plasma (all porous volume is filled with plasma), all activity with respect to movement of liquid sample through plasma extractor assembly ceases because all capillaries of absorbent probe 112, separator 114, and absorbent reservoir 116 are full at this point.

Accordingly, an example of the present disclosure provides a method for extraction of plasma, comprising wicking a volume of a liquid sample from a source through a first capillary action, wicking the liquid sample to separator 114 through a second capillary action, with separator 114 generating a volume of a plasma, and finally, wicking the plasma from separator 114 through a third capillary action instantiated by differences in hydrophilic nature between absorbent reservoir 116 and separator 114.

It should be understood that the dried plasma stored within absorbent reservoir 116 may later be processed by detectors designed for analysis, non-limiting, non-exhaustive listing of examples of which may include immunoassay, Liquid Chromatography-Mass Spectrometry (LCMS), Ultraviolet (UV) visible detector, High performance Liquid Chromatography (HPLC), fluorescence detector, and or Amino acid applications, immunoassay, etc. The extraction of dried plasma from absorbent reservoir 116 may be accomplished by any well-known manner, including acquiesce (re-dissolve plasma), organic (placing dried plasma into an organic solvent such as methanol), or other types of extractions.

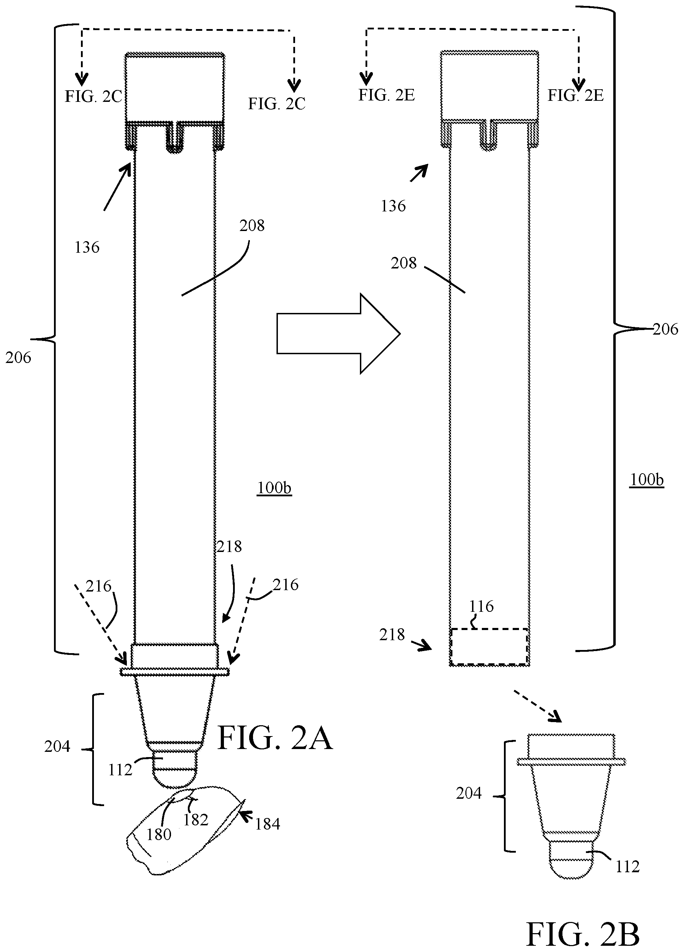

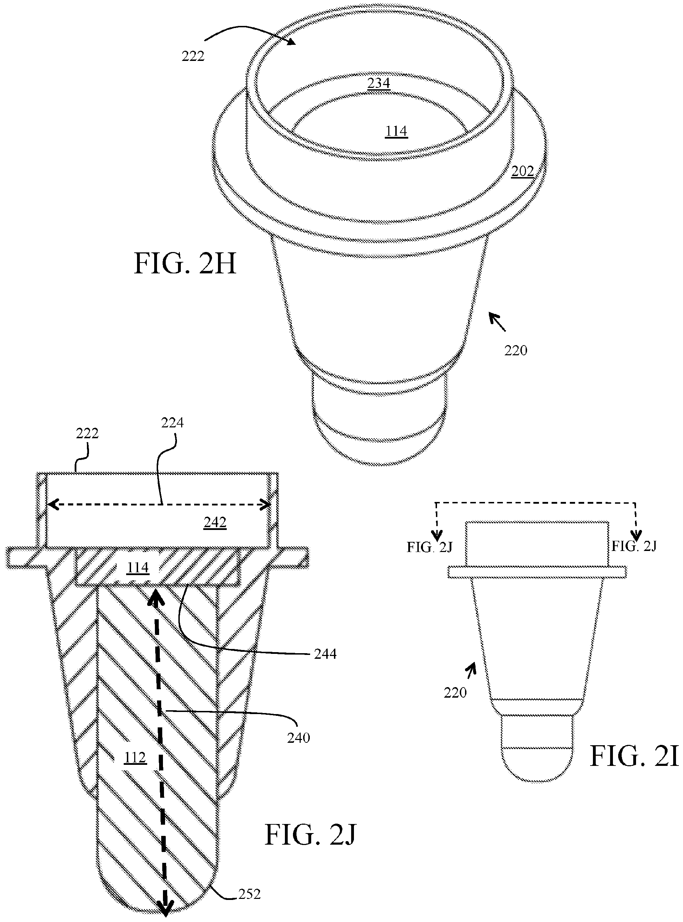

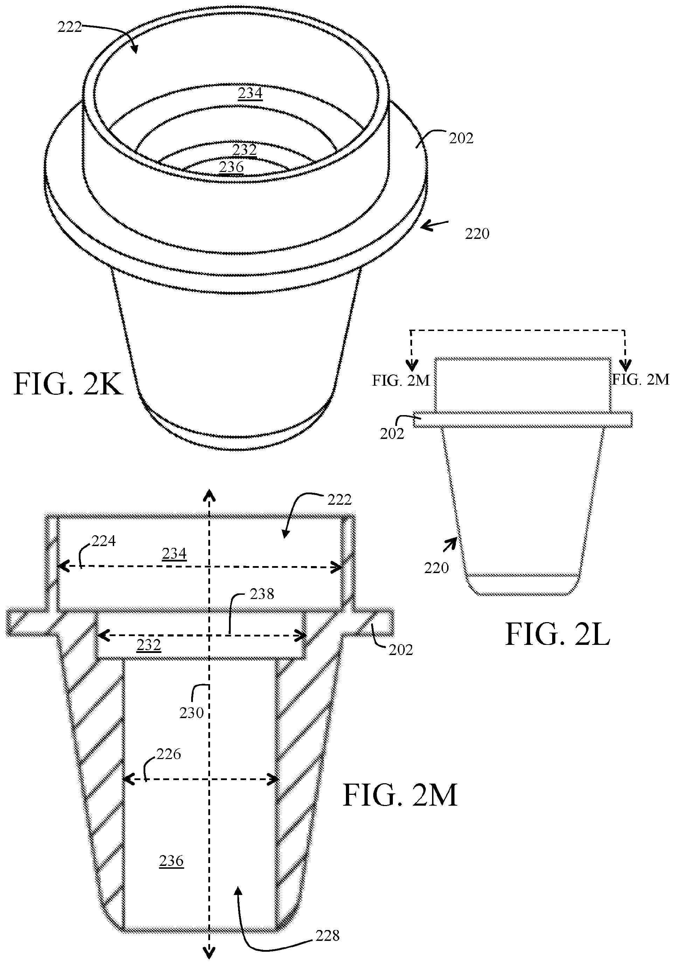

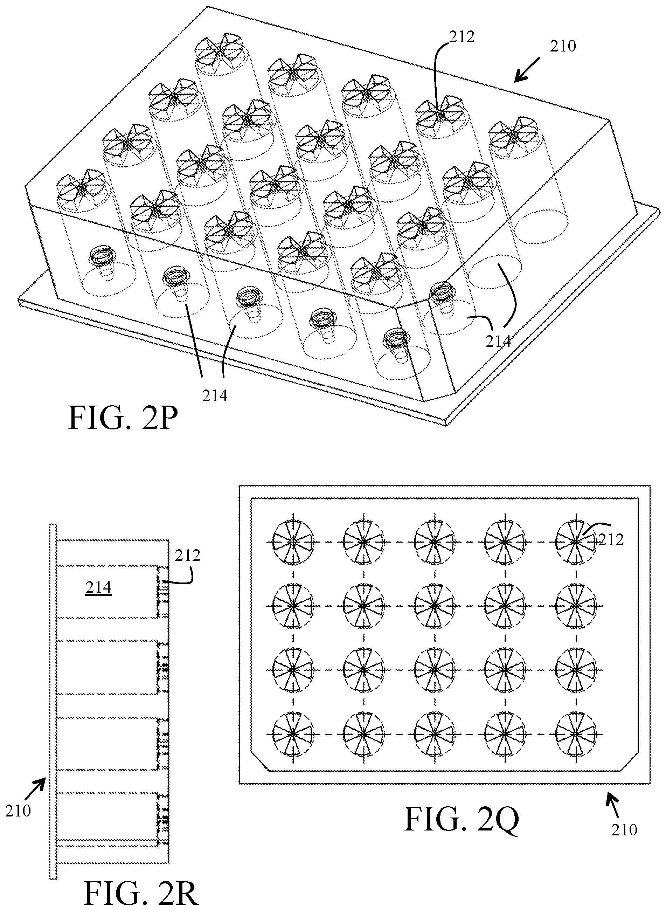

FIGS. 2A to 2R are non-limiting, exemplary illustrations of various views of a plasma extraction device for extraction of plasma from a liquid sample in accordance with one or more embodiments of the present invention. Plasma extraction device 100b illustrated in FIGS. 2A to 2R includes similar corresponding or equivalent components, interconnections, functional, operational, and or cooperative relationships as plasma extraction device 100a that is shown in FIGS. 1A to IN, and described above. Therefore, for the sake of brevity, clarity, convenience, and to avoid duplication, the general description of FIGS. 2A to 2R will not repeat every corresponding or equivalent component, interconnections, functional, operational, and or cooperative relationships that has already been described above in relation to plasma extraction device 100a that is shown in FIGS. 1A to IN.

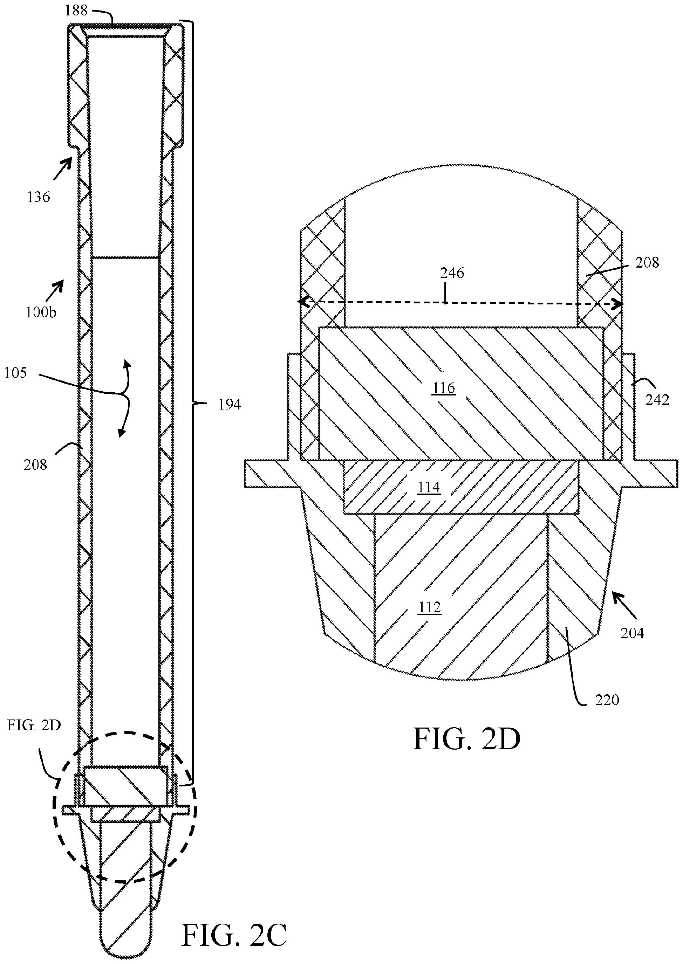

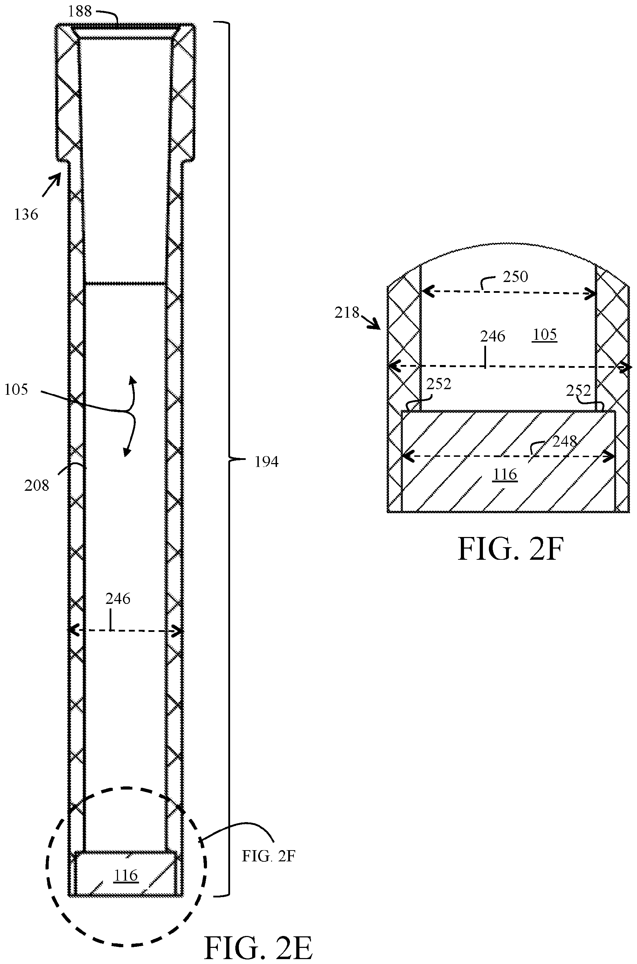

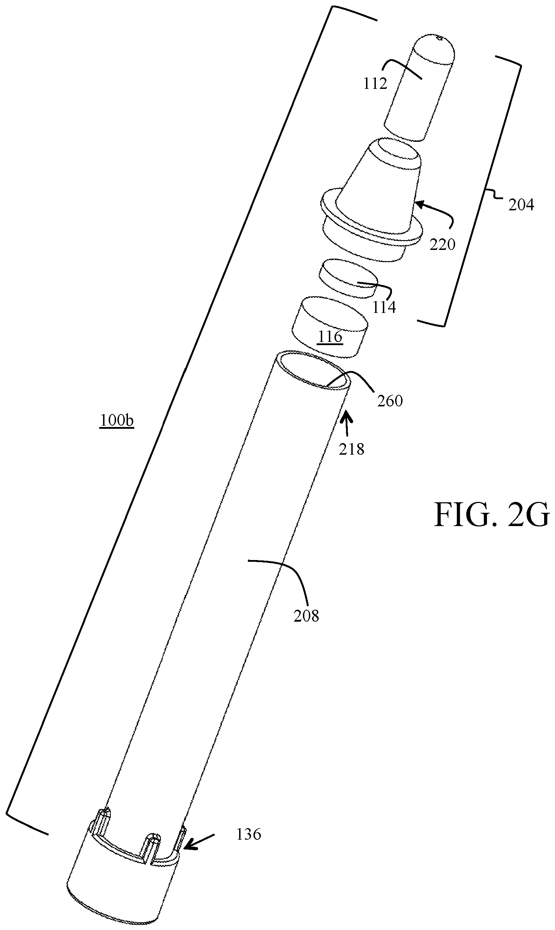

As illustrated in FIGS. 2A to 2R, in this non-limiting, exemplary embodiment, absorbent probe 112 and separator 114 are accommodated or housed within a plasma extractor module 204 that is detachably associated with a handler assembly 206 that includes a handler 208 and absorbent reservoir 116. As best shown in sectional views FIGS. 2C, 2E, and 2G handler 208 is identical to first section 194 (including first distal end 136 and top openings 188) of handler 132 of handler assembly 130 with the exception of the lower or second distal end 218 and bottom opening 260. This way, plasma extraction device 100b may also be used and handled for automated processing by the same well known automated liquid sample handling instruments.

As best illustrated in FIGS. 2A, users may dip absorbent probe 112 of plasma extraction module 100b into a liquid source 180 (for example, from cut 182 of finger 184) to extract plasma from liquid sample, and optionally, place back plasma extraction device 100b securely within tube 102, enabling the liquid sample plasma to dry via vent holes 108 on tube 102 (FIG. 1A). As further detailed below, plasma extraction module 204 is later detached as shown in FIG. 2B and discarded. The remaining plasma loaded absorbent reservoir 116 within handler assembly 206 is then processed by well known automated liquid sample handling instruments.

When plasma extraction module 204 is attached and fully assembled as illustrated in FIGS. 2A, 2C, and 2D, all members of the plasma extractor assembly (i.e., absorbent probe 112, separator 114, and absorbent reservoir 116) have full, surface-to-surface contact (best illustrated in FIG. 2D). FIGS. 2B, 2E, and 2F illustrate plasma extractor module 204 detached from handler assembly 206, including the remaining retained absorbent reservoir 116.

FIG. 2G is a non-limiting exemplary exploded view illustration of the various components of the plasma extraction device 100b in accordance with one or more embodiments of the present invention. The exploded view shown in FIG. 2G illustrates disassembled, separated components that show the cooperative working relationship, orientation, positioning, and exemplary manner of assembly of the various components of plasma extraction device 100b in accordance with one or more embodiments of the present invention, with each component detailed below.

Plasma extractor module 204 is detachable friction-fit (or compression or press fit) secured onto handler 208. Plasma extractor module 204 includes engagement structural wall 242 with an inner diameter 224 (FIG. 2J) that may be slightly larger than outer diameter 246 (FIG. 2D) of lower distal end 218 of handler 208, enabling plasma extractor module 204 to be friction-fit (or compression-fit) secured on lower distal end 218 of handler 208.

As further best illustrated in FIGS. 2E and 2F, absorbent reservoir 116 is also friction or press fit secured within lower distal end 218 (inside bottom opening 260 shown in FIG. 2G) of handler 208. Lower distal end 218 includes an inner diameter 248 that is slightly longer than an inner diameter 250 of the rest of handler 208 to accommodate and house absorbent reservoir 116. The differential in the inner diameter sizes 248 and 250 result in a step structure 252 that prevents absorbent reservoir 116 from falling back into hollow portion 105 of handler 208. When plasma extractor module 204 is detached and separated from handler 208 as shown in FIG. 2B, absorbent reservoir 116 is still securely retained within handler 208 as best illustrated in FIGS. 2B, 2E, and 2F, while the whole of plasma extractor module 204 (which includes separator 114 and absorbent probe 112) is detached.

FIGS. 2D and 2H to 2M provide detailed views of the plasma extractor module, with FIGS. 2K to 2M illustrating plasma extractor module housing, but with absorbent probe 112 and separator 114 removed. As illustrated, plasma extractor module 204 includes and houses both separator 114 and absorbent probe 112, but not absorbent reservoir 116. Plasma extractor module 204 is comprised of a housing 220 configured generally similar to a frustum of a right circular cone. Housing 220 includes a top opening 222 with a wider diameter 224, and a bottom opening 228 with a narrower diameter 226, forming a through-opening or hollow body portion along an inner longitudinal axis 230.

Housing 220 of plasma extractor module 204 includes a compartment 232 that securely houses separator 114, with absorbent probe 112 frictional secured within chamber 236 of housing 220 through bottom opening 228. Compartment 232 is defined by wider upper chamber 234 that receives lower distal end 218 of handler 208 through top opening 222, and the narrower lower chamber 236 defined by bottom opening 228. Compartment 232 has a diameter 238 that is longer than diameter 226 of bottom opening 228, but shorter than upper chamber diameter 234. Housing 220 further includes an external, outer circumferentially extending flange 202 that may be used to push out (shown by arrows 216 in FIG. 2A) and detach plasma extractor module 204 from handler 208.

Absorbent probe 112 is friction-fit secured within bottom opening 228 (inside chamber 236) of housing 220 of plasma extractor module 204. Absorbent probe 112 has sufficient height 240 to allow a first (or probing end) 252 to extend out from bottom opening 228 of housing 220 of plasma extractor module 204, with a second (or lodging end) 244 of absorbent probe 112 physically contacting separator 114, as illustrated in FIG. 2J.

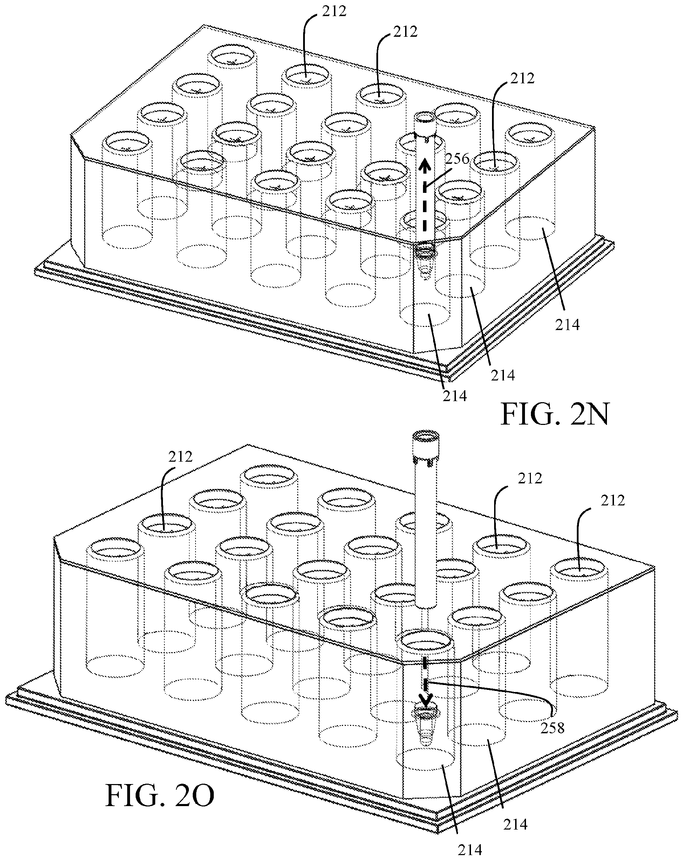

The actual operation (i.e., fluid dynamics) for loading absorbent reservoir 116 with plasma is the same as plasma extraction device 100a. Once loaded, device 100b may be moved and inserted into storage compartments 214 in tray 210 illustrated in FIGS. 2N to 2R. Thereafter, tray 210 illustrated in FIGS. 2N to 2R, may than be aligned with moving arms or gripping mechanisms of well known automated plasma analysis instruments, where plasma extraction device 100b may be lifted out from the inserted storage compartment 214 within tray 210 as shown by arrow 256. In this non-limiting exemplary instance, once lifted up to be moved out by handler 208, annular flange 202 of plasma extractor module 204 contacts or catches edges (wings or flaps) 212 of storage compartments 214 of tray 210, where module 204 is disengaged from handler assembly 206. Once disengaged, as illustrated, plasma extractor module 204 gets trapped and falls back into and remains inside storage compartment 214 (as shown by arrow 258), with absorbent reservoir 116 still frictionally retained and remaining in hander 208 and free from the remaining extractor module 204 as best shown in FIG. 2B. In other words, flaps 212 operate as trap doors that allow easy insertion of handler assembly 206 and associated plasma extractor module 204 into storage compartment 214, but block removal of plasma extractor module 204 by pushing against annular flange 202 along direction shown by arrows 216 (FIG. 2A), dislodging extractor module 204 as shown in FIG. 2O. In other words, the force of the push of flaps 212 against flange 202 is sufficiently strong to overcome the frictional hold of plasma extractor module 204 together with handler assembly 208 to thereby release plasma extractor module 204 from handler 208. Thereafter, plasma from absorbent reservoir 116 may be extracted by solvents via well-known irrigation and aspiration methodologies used by well-known automated plasma analysis instruments. It should be noted that the ejection of plasma extraction module 204 may optionally be accomplished directly by automated plasma analysis instruments (as above) and need not use tray 210 or flaps 212 illustrated in FIGS. 2N to 2R.

It should be noted that plasma extraction device 100a shown in FIGS. 1A to IN is adapted to allow complete automation through the body of the device 100a itself. It would allow for a "two-stage" release of both probe 112 and then reservoir 116. It is reservoir 116 that will be analyzed (in most cases), and the ejection of reservoir 116 allows translocational freedom during the subsequent agitation events. This freedom of movement allows for more efficient extractions when vortexing and sonicating the sample. This type of workflow would be more obvious to current users of Dried Blood Spot (DBS) cards because in those cases, a subpunch of the DBS card is dropped into the well for extraction.

On the other hand, reservoir 116 in relation to plasma extraction device 100b shown in FIGS. 2A to 2R is not ejected. In this case, the extraction takes place by aspiration and dispensing of extracting solution through reservoir 116. This is much more amenable to current automation approaches. However, it may suffer from less efficient extraction due to the absence of strong vortexing and sonication.

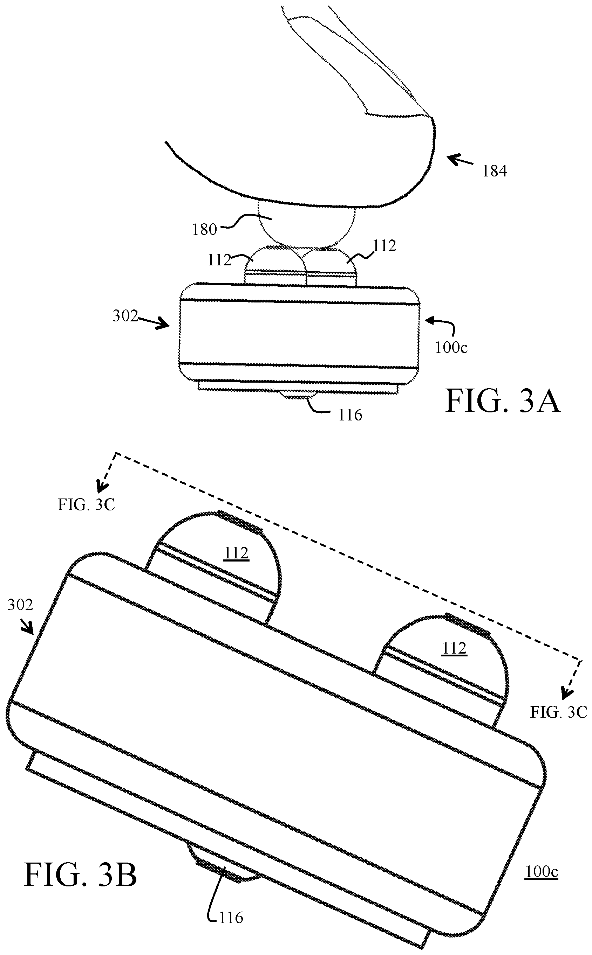

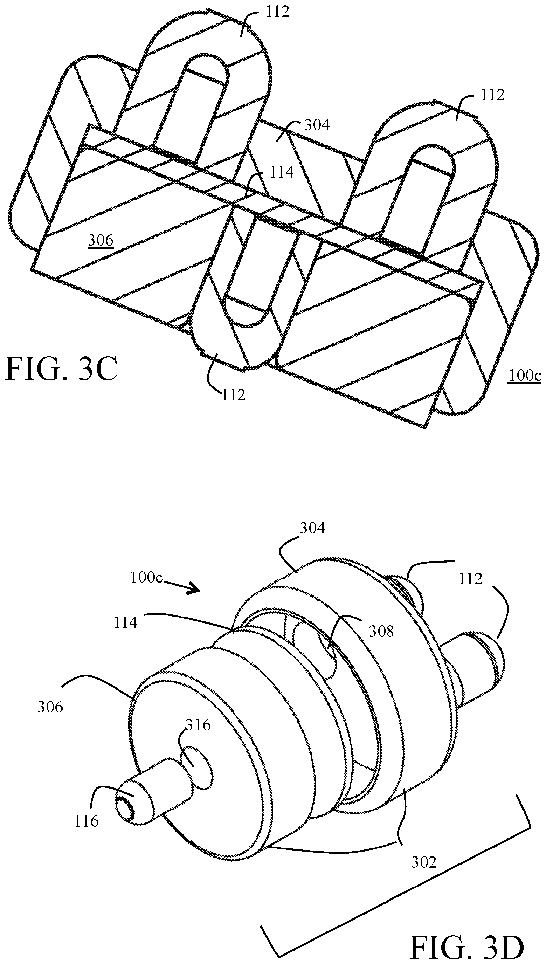

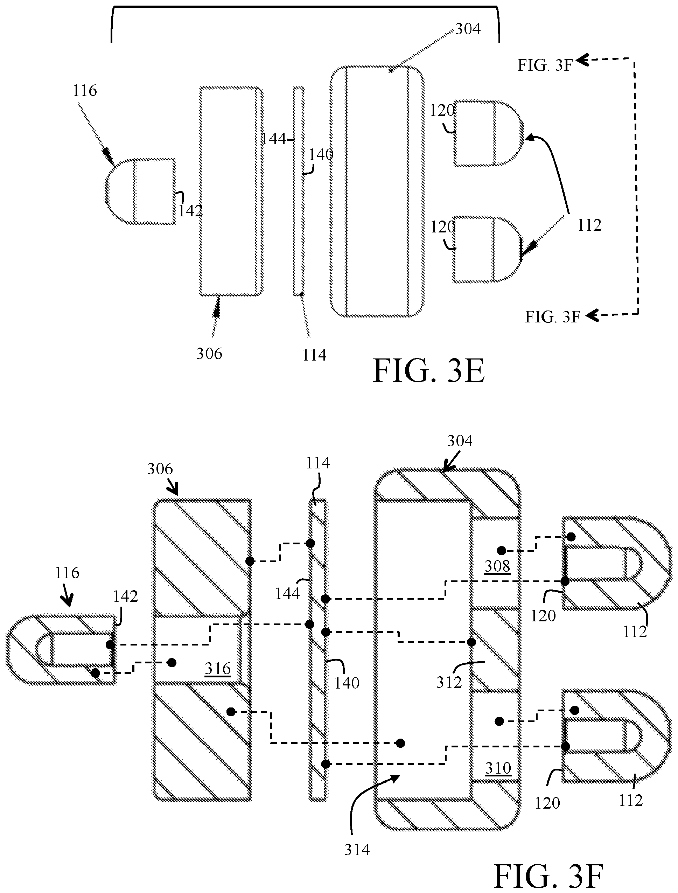

FIGS. 3A to 3F are non-limiting, exemplary illustrations of various views of a plasma extraction device for extraction of plasma from a liquid sample in accordance with one or more embodiments of the present invention. Plasma extraction device 100c illustrated in FIGS. 3A to 3F includes similar corresponding or equivalent components, interconnections, functional, operational, and or cooperative relationships as plasma extraction devices 100a and 100b that are shown in FIGS. 1A to 2R, and described above. Therefore, for the sake of brevity, clarity, convenience, and to avoid duplication, the general description of FIGS. 3A to 3F will not repeat every corresponding or equivalent component, interconnections, functional, operational, and or cooperative relationships that has already been described above in relation to plasma extraction devices 100a and 100b that are shown in FIGS. 1A to 2R.

As illustrated in FIGS. 3A to 3F, in this non-limiting embodiment, plasma extraction device 100c is comprised of a small, compact form-factor having a set of absorbent probes 112 in contact with a fluid sample 180 for loading plasma onto absorbent reservoir 116.

As illustrated in FIGS. 3A to 3F, a housing assembly 302 is provided that is comprised of first and second pieces 304 and 306. First piece 304 of housing assembly 302 accommodates two absorbent probes 112 and separator 114. Second piece 306 of housing 302 accommodates a single absorbent reservoir 116.

Absorbent probes 112 is friction (or compression) fit and secured within corresponding number of through-openings 308 and 310 on a first side 312 of first piece 304 while separator 114 is housed within cavity or compartment 314 thereof. Absorbent reservoir 116 is also friction (or compression) fit and detachably secured within corresponding number of through-openings 316 on second piece 306.

First piece 304 has a larger size compared to the smaller sized second piece 306, allowing the smaller sized second piece 306 to frictionally (or compression) fit (but be detachably) secured within compartment 314 of first piece 306. This arrangement allows side 140 of separator 114 to be pressed against sides 120 of absorbent probes 112, and side 142 of absorbent reservoir 116 to be pressed against side 144 of separator 114. The actual operation (i.e., fluid dynamics) for loading absorbent reservoir 116 with plasma is the same as plasma extraction devices 100a and 100b. Once loaded with plasma, absorbent reservoir 116 may be physically removed and extracted out of opening 316 of second piece 306 and dry-transferred for assay. It should be noted that the number, size, and shape of absorbent probes 112, separator 114, and absorbent reservoirs 116 may be varied, but in general, larger number or size of absorbent probes 112 would be required to extract plasma from a liquid sample compared to the number of absorbent reservoir 116 used.

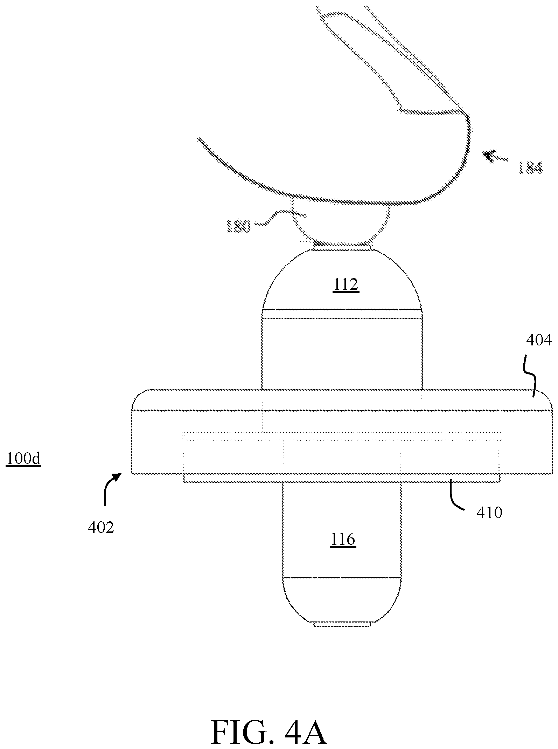

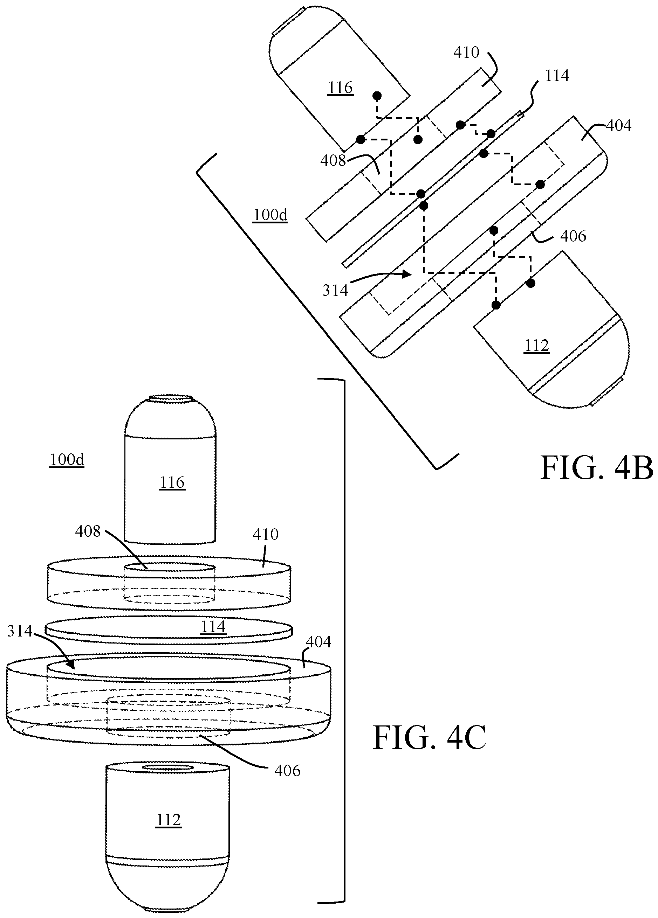

FIGS. 4A to 4C are non-limiting, exemplary illustrations of various views of a plasma extraction device for extraction of plasma from a liquid sample in accordance with one or more embodiments of the present invention. Plasma extraction device 100d illustrated in FIGS. 4A to 4C includes similar corresponding or equivalent components, interconnections, functional, operational, and or cooperative relationships as plasma extraction devices 100a, 100b, and 100c that are shown in FIGS. 1A to 3F, and described above. Therefore, for the sake of brevity, clarity, convenience, and to avoid duplication, the general description of FIGS. 4A to 4C will not repeat every corresponding or equivalent component, interconnections, functional, operational, and or cooperative relationships that has already been described above in relation to plasma extraction devices 100a, 100b, 100c that are shown in FIGS. 1A to 3F.

As illustrated in FIGS. 4A to 4C, in this non-limiting embodiment, plasma extraction device 100d has a housing assembly 402 that accommodates a single large absorbent probe 112 on first piece 404 secured within a single, larger through opening 406 compared to a smaller sized absorbent reservoir 116 and its corresponding smaller opening 408 on a smaller second piece 410. As with plasma extracting device 100c, all components are friction (press) fit together and operate the same, and further, the number, size, and shape (e.g., polygonal) of absorbent probe 112, separator 114, absorbent reservoir 116 may vary. The actual operation (i.e., fluid dynamics) for loading absorbent reservoir 116 with plasma is the same as plasma extraction devices 100a, 100b, and 100c.

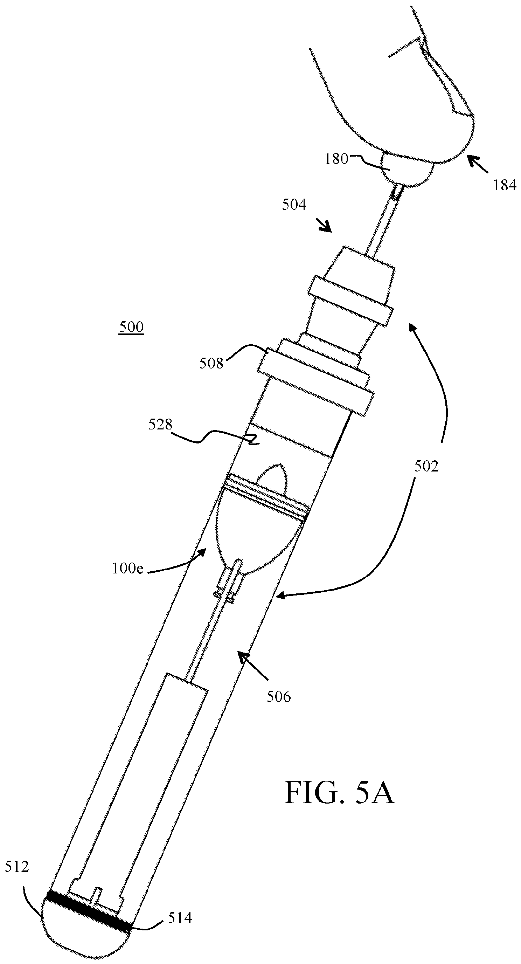

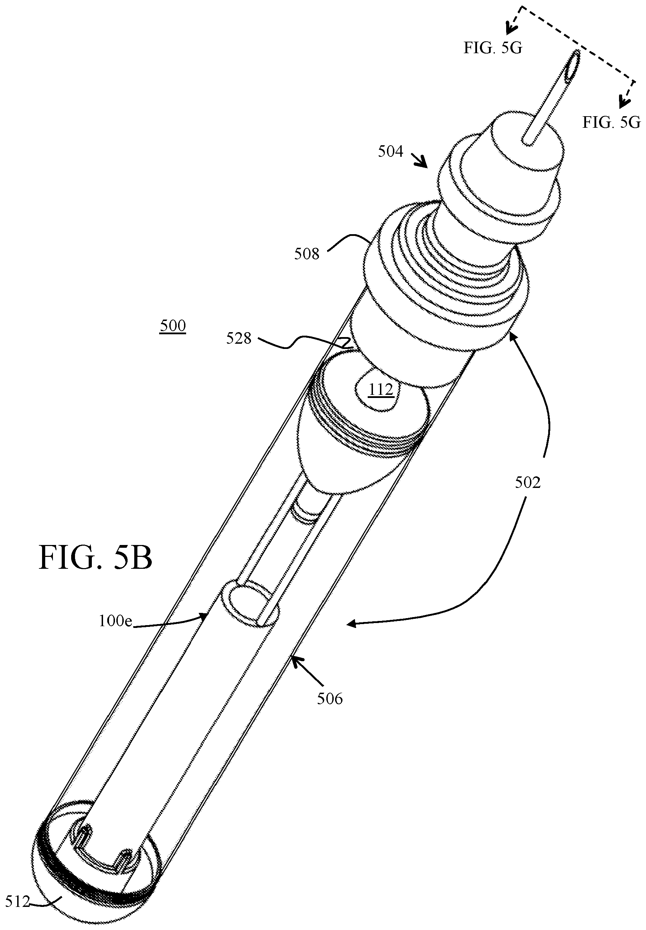

All of the embodiments shown and described above in relation to FIGS. 1A to 4C may be broadly categorized as "standalone" and "passive" devices (i.e., plasma extraction devices) that operate based on capillary action as the main motive force. The remaining embodiments detailed below in relation to FIGS. 5A to 8D may be broadly categorized as "active" devices (i.e., plasma extraction assemblies) in that the motive force to generate plasma is by induced pressure differential aided by various types of fluid flow facilitators, in addition to capillary action.

In general, the time it takes to wick fluid sample (e.g., blood) from source 180 (e.g., from cut 182 of finger 184) and onto absorbent probe 112 driven by capillary action alone is a long duration. For example, it may potentially take about 15 seconds of direct, physical contact time between absorbent probe 112 and cut 182 to wick about 60 µL of fluid sample onto absorbent probe 112. The duration of 15 second may create discomfort and pain for the patient. On the other hand, use of a fluid flow facilitator detailed below in relation to FIGS. 5A to 8D reduce the 15 seconds duration of physical contact time between absorbent probe 112 and cut 182 to about 1 second, which allows quick removal of the entire plasma extraction assemblies away from patient. Accordingly, the use of plasma extraction assemblies detailed in FIGS. 5A to 8D improve patient comfort, reduce pain, and improve overall patient experience. That is, the addition of fluid flow facilitators in plasma generation overcomes the long sample collection times for absorbent probe 112 by nearly instantly placing and storing fluid within fluid flow facilitators and in contact with probe 112 without the need for probe 112 to be in full contact with patient while it continues to wick fluid. This way, probe 112 may wick fluid from within the fluid flow facilitator while the entire unit (i.e., plasma extraction assembly) is moved away from and no longer in contact with the patient, allowing superior patient experience.

FIGS. 5A to 8D disclose various types of plasma extraction assemblies 500, 600, 700, and 800 that exemplarily show the use of a plasma extraction device 100e, which is identical to plasma extraction device 100a with the exception of an additional o-ring 530 (detailed below). However, any one of the other plasma extraction devices 100b to 100d detailed above in relation to FIGS. 1A to 4C may also be used instead, but with added appropriate sealing member (if any) to trap fluid sample in contact with absorbent probe 112 within fluid flow facilitator (as detailed below). Accordingly, the use of plasma extraction device 100e (identical to device 100a with the added o-ring 530) in plasma extraction assemblies 500, 600, 700, and 800 shown in respective FIGS. 5A to 8D is only an example and for discussion purposes and should not be limiting.

FIGS. 5A to 5H are non-limiting, exemplary illustration of various views of a plasma extraction assembly for extraction of plasma from a liquid sample in accordance with one or more embodiments of the present invention. Plasma extraction assembly 500 illustrated in FIGS. 5A to 5H includes a plasma extraction device 100e with similar corresponding or equivalent components, interconnections, functional, operational, and or cooperative relationships as plasma extraction devices 100a, 100b, 100c, and 100d that are shown in FIGS. 1A to 4C, and described above. Therefore, for the sake of brevity, clarity, convenience, and to avoid duplication, the general description of FIGS. 5A to 5H will not repeat every corresponding or equivalent component, interconnections, functional, operational, and or cooperative relationships that has already been described above in relation to plasma extraction devices 100a, 100b, 100c, and 100d that are shown in FIGS. 1A to 4C.

As illustrated in FIGS. 5A to 5H, in this non-limiting exemplary embodiment, plasma extraction assembly 500 is comprised of a plasma extraction device 100e that is associated with a fluid flow facilitator 502. Plasma extraction device 100e is identical to plasma extraction device 100a, with the exception of an added sealing member (o-ring seal 530) detailed below.

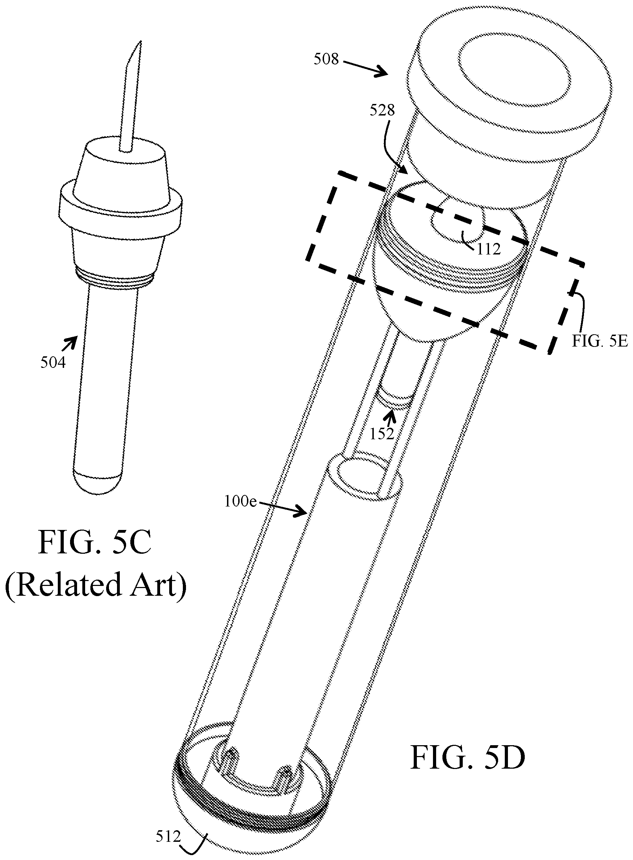

In general, in this non-limiting exemplary instance, fluid flow facilitator 502 is comprised of a dual-cannula needle assembly 504 and an air evacuated container assembly 506. Dual-cannula needle assembly 504 (best illustrated in FIG. 5C) and its use with conventional evacuated tube glass (not shown) to draw blood is well known and detailed in numerous publications such as for example U.S. Patent 4,980,297 to Haynes et al.

In operation for extraction of blood plasma, one cannula of the needle assembly 504 may be poked into the subject's finger 184 (or vein) and piercing stopper 508 (FIG. 5D) of evacuated container assembly 506 with the other cannula of the needle assembly 504, thereby establishing fluid communication between fluid sample 180 (or vein) and interior space 528 of tube assembly 506. Due to the lower pressures within the evacuated tube assembly 506, fluid is drawn from the subject into tube assembly 506, and trapped within space 528. Thereafter, the entire until may be separated from the subject, while the first absorbent member 112 wicks fixed, predetermined quantity of liquid sample 180 (now inside chamber 528), with plasma generated as detailed above in relation to FIGS. 1A to 4C.

It should be noted that although the illustrated plasma extraction assembly 500 could operate without an absorbent probe 112, the use of probe 112 eliminates orientation requirement for the plasma extraction device itself. That is, once absorbent probe 112 is full, plasma extraction device may be held in any orientation and the capillary effect will still continue, providing a better patient experience. If absorbent probe 112 was removed, and the plasma extraction device was used to collect blood and then oriented so the separator was up, the blood would drip off away from separator due to gravity and never interact with separator. This would create an undesired lengthy duration orientation requirement. In the absence of absorbent probe 112 plasma extraction device may need to be held in the correct orientation for approximately 3-5 minutes.

Plasma extraction device 100b may be used instead of the illustrated plasma extraction device 100e. In fact, for example, annular flange 202 of plasma extraction module 204 of plasma extraction device 100b illustrated in FIGS. 2A to 2R may also function as "o-ring sealant" to trap fluid sample within fluid flow facilitator in contact with absorbent probe 112 without the use of any o-ring or sealing member. Thereafter, when pulled to be removed from tube assembly 506, plasma extraction module 204 may simply be left within container assembly 506 due to friction force between flange 202 and inner circumferential surface 534 of container assembly 506. The rest of handler assembly 206 with its retained absorbent reservoir 116 may be pulled out to be processed as described above.

As another example, the use of plasma extraction devices 100a, 100b, and 100e with handlers is not necessary for the separation of plasma, but offer the benefit of easy, automated detachment process of the plasma sample (absorbent reservoir 116) from the rest of the plasma extraction device. Without the handler (e.g., using embodiments disclosed in FIGS. 3A to 4C or simply using a separator and an absorbent reservoir only), there would still be a requirement of some sort of manual disassembly and handling of the fluid sample loaded absorbent reservoir 116, which would make the entire process significantly less efficient in a clinical setting. The handlers enable well known automated processing of absorbent reservoir 116 by well known assay instruments.

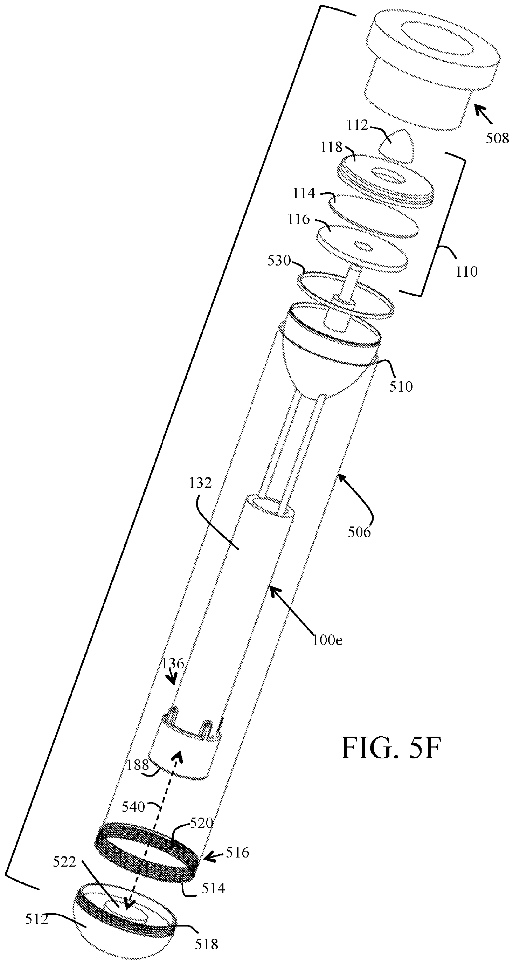

FIG. 5D is a non-limiting exemplary illustration of the plasma extraction device and container assembly in accordance with one or more embodiments of the present invention. FIG. 5F is a non-limiting exemplary exploded view illustration of the plasma extraction device and container assembly show in FIG. 5D in accordance with one or more embodiments of the present invention. The exploded view shown in FIG. 5F illustrates disassembled, separated components that show the cooperative working relationship, orientation, positioning, and exemplary manner of assembly of the various components of plasma extraction device 100e and container assembly 506 in accordance with one or more embodiments of the present invention.

As illustrated in FIGS. 5A to 5H, fluid flow facilitator 502 is comprise of a container assembly 506 in accordance with one or more embodiments of the present invention that may be configured as a tube assembly with air evacuated from within to create reduced pressure inside container (or tube) assembly 506. That is, the interior of container assembly 506 has an absolute internal pressure that is less than atmospheric pressure. Container assembly 506 includes a first detachable closure 508 (conventional pierce-able-stopper) to airtight close (or hermetically seal) a first open end 510 of container assembly 506. Further included is a second detachable closure 512 to airtight close (or hermetically seal) a second open end 514 of container assembly 506.

First detachable closure 508 is seal-punctured to draw fluid inside container assembly 506 driven by pressure differential between inside and outside container assembly 506. Second detachable closure 512 is used to enable access into container assembly 506 to position plasma extraction device 100e within container assembly 506 as illustrated, and remove plasma extraction device 100e once extraction of liquid sample 180 is complete, without contacting or having to remove dual-cannula needle assembly 504. In other words, second detachable closure 512 operates as a sealed cap that enables removal of the plasma extraction device 100e from the far end (lower distal end) 516 of container assembly 506. That is, once plasma is generated, sealed cap 512 may be removed to remove the entire plasma extraction device 100e.

Second detachable closure 512 has female threading 518 (best shown in FIG. 5F) that hermetically fasten onto male-threaded 520 of second distal end 516 of container assembly 506, near second opening 514. In other words, the mechanical connection between second detachable closure 512 and second distal ends 516 of container assembly 506 is simply a threaded seal that enable hermetical sealing of container assembly 506. It should be noted that threaded seal fastening schemes are well known, for example, they may comprise of rubber-threaded seals where the threads are comprised of rubber sealant. Also, second detachable closure 512 does not have to be shaped as a dome (semi-hemispheric). Further, other mechanisms (other than male-female threading) may be used to detachably and hermetically fasten second detachable closure 512 to container assembly 506, non-limiting examples of which may include, for example, snap-fit mechanisms that provide hermetic sealing.

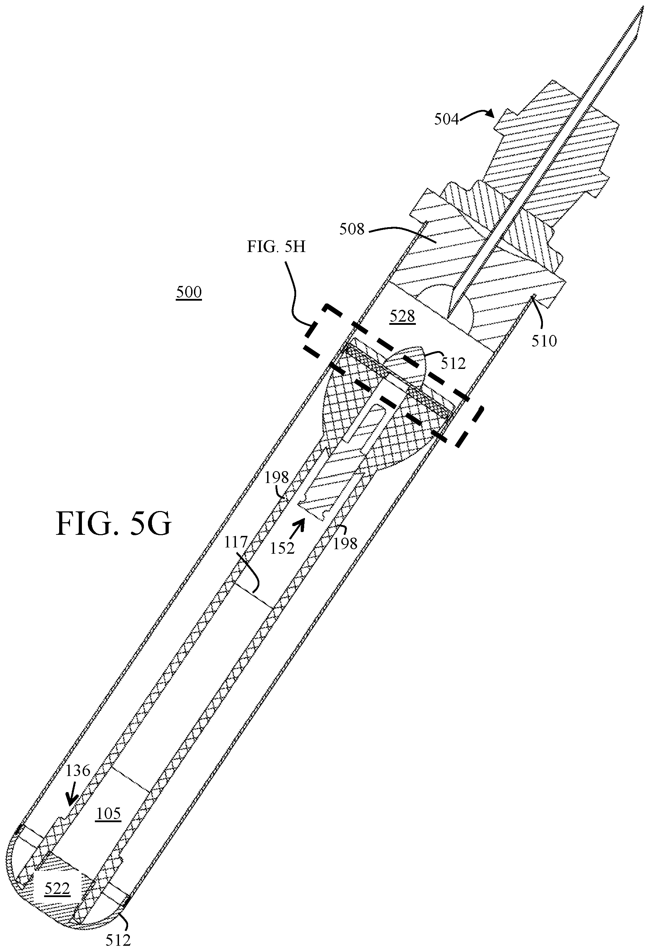

As further illustrated, second detachable closure 512 further includes a post or support 522 (best shown in FIGS. 5F and 5G) that is received (shown by arrow 540) within hollow chamber 505 through top opening 188 of plasma extraction device 100e. Support 522 maintains position and supports alignment of plasma extraction device 100e within container assembly 506.

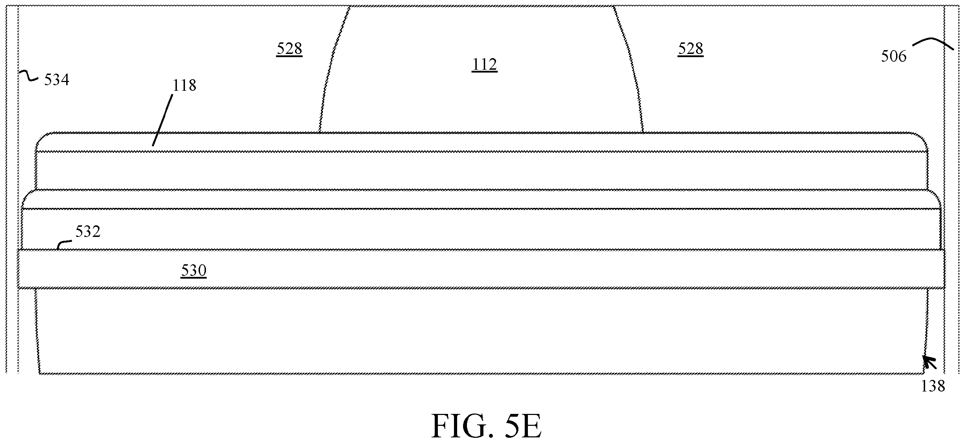

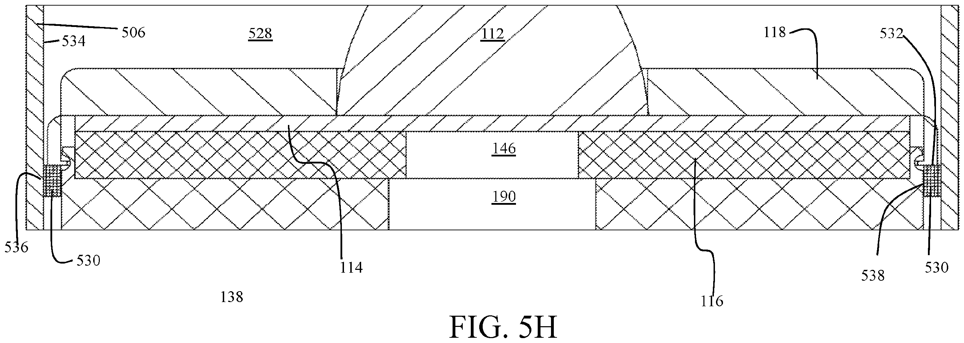

As best illustrated in FIGS. 5E, 5F, and 5H, in this non-limiting exemplary instance, plasma extraction device 100e includes an outer o-ring seal 530 that hermetically seals and isolates interior space 528 (FIG. 5A) of container assembly 506 between the first and second openings 510 and 514 from the rest of interior of container assembly 506. This way, once first detachable closure 508 is seal-punctured by dual cannula needle 504 to draw fluid inside container assembly 506 driven by pressure differential between inside and outside container assembly 506, fluid is collected and is retained or trapped within first interior space 528, in full contact with and flooded over absorbent probe 112. O-ring 530 (FIG. 5H) helps create a better seal around the plasma extractor module 110 to prevent fluid (e.g., blood) from leaking into the rest of the container assembly.

Inner diameter side 538 of o-ring seal 530 is associated or contacts lower distal 138 end of handler 132 of handler assembly 130, underneath edge 532 of housing 118 of plasma extractor module 110 while outer diameter side 536 of o-ring seal 530 is associated or contacts an inner circumference 534 of container assembly 506, thus preventing or blocking fluid accumulated within interior space 528 of container assembly 506 from leaking out thereof.

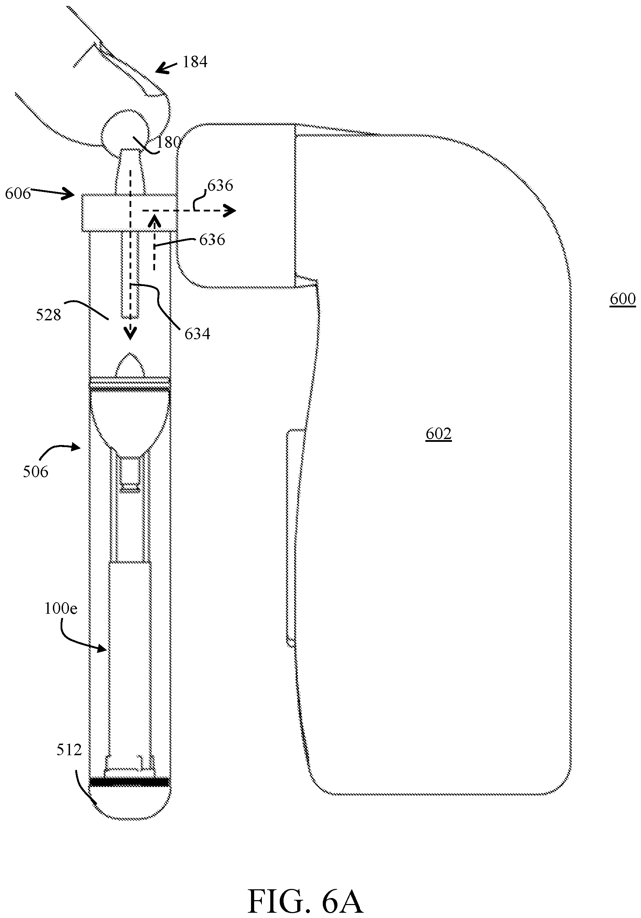

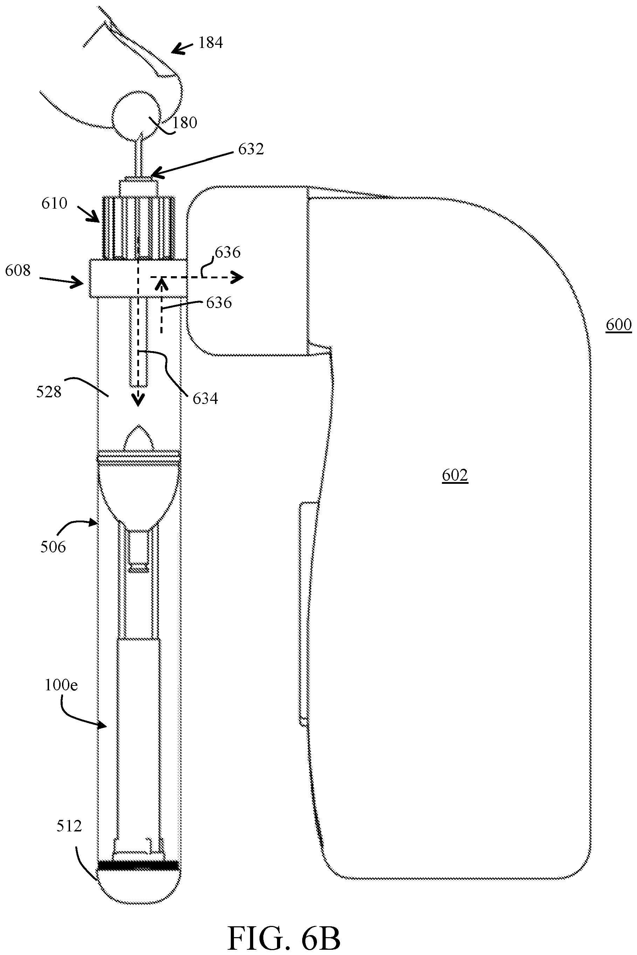

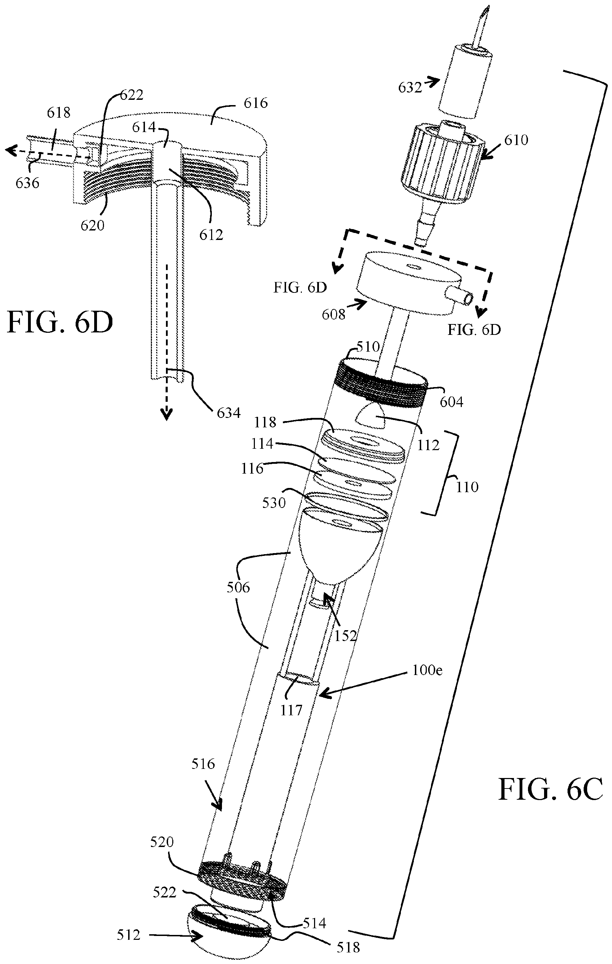

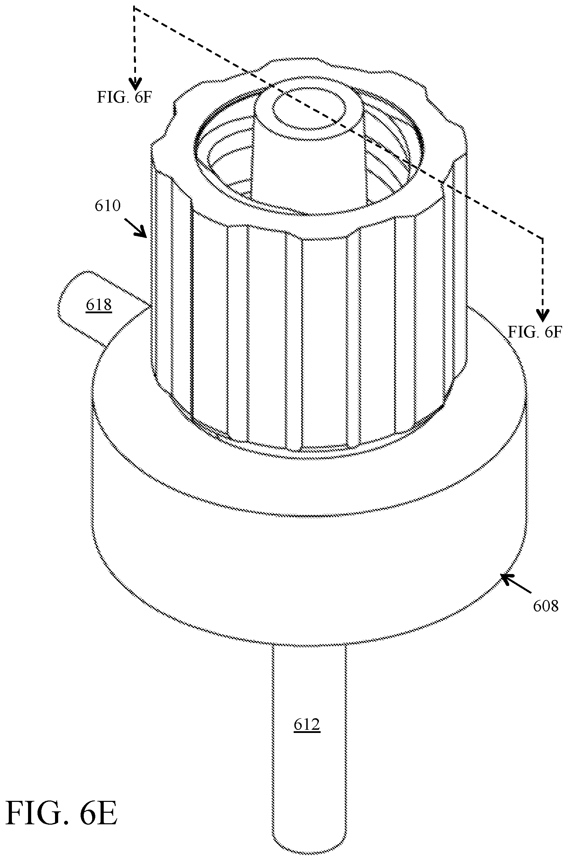

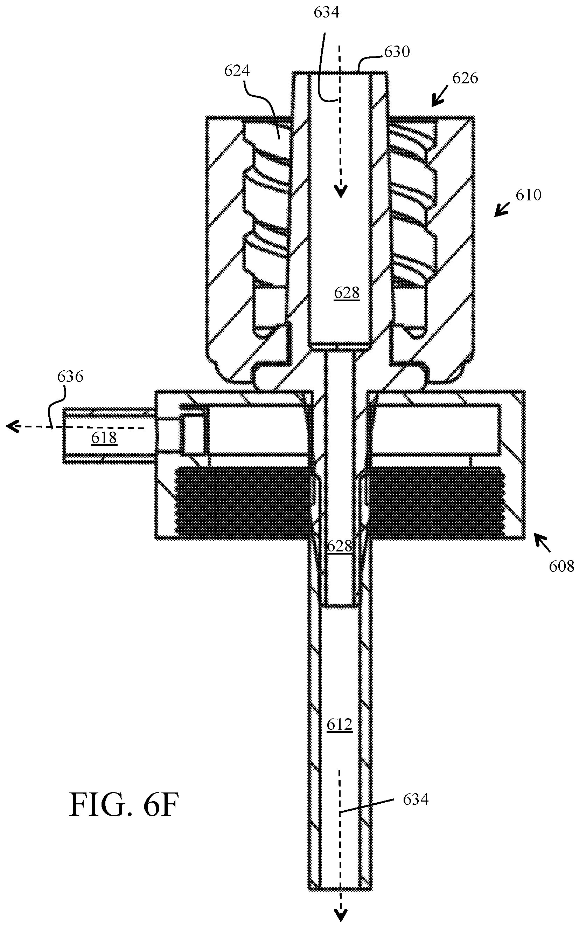

FIGS. 6A to 6F are non-limiting, exemplary illustration of various views of a plasma extraction assembly for extraction of plasma from a liquid sample in accordance with one or more embodiments of the present invention. Plasma extraction assembly 600 illustrated in FIGS. 6A to 6F includes similar corresponding or equivalent components, interconnections, functional, operational, and or cooperative relationships as plasma extraction assembly 500 shown in FIGS. 5A to 5H, and described above. Therefore, for the sake of brevity, clarity, convenience, and to avoid duplication, the general description of FIGS. 6A to 6F will not repeat every corresponding or equivalent component, interconnections, functional, operational, and or cooperative relationships that has already been described above in relation to plasma extraction assembly 500 and plasma extraction devices 100a, 100b, 100c, 100d, and 100e that are shown in FIGS. 1A to 5H.

In this non-limiting, exemplary embodiment, plasma extraction assembly 600 provides a different source of vacuum from that of plasma extraction assembly 500. Accordingly, in this non-limiting, exemplary embodiment fluid flow facilitator 502 is comprised of a pressure differential generator 602 in addition to container assembly 506. For example, plasma extraction assembly 600 may use a well-known conventional vacuum or pressure differential generator 602 (for example, by INNOVATIVE MED TECH™ known as INNOVAC QUICK-DRAW™) to evacuate air from container assembly 506. As the pressure within container assembly 506 drops due to flow of air out of container assembly 506 (in the direction shown by arrow 636), fluid sample is pulled into container assembly 506, flooding over absorbent probe 112 due to the pressure differential in the direction shown by arrow 634. In the non-limiting, exemplary instance shown in FIG. 6C, container assembly 506 need not be hermetically sealed and is comprised of first open end 510 that has threading 604 that fastens onto a well-known first detachable adapter 606.

FIGS. 6A to 6F use a modified first detachable adapter 608 that may be associated with a well-known luer lock adapter 610 to receive externally connected devices such as an invasive probe 632. First detachable adapter 608 is similar to a conventional first detachable adapter 606, but with the exception that first detachable adapter 608 is comprised of a fluid inlet 612 having an opening 614 that is flush with a generally flat top surface 616.

As illustrated in FIGS. 6A to 6F, plasma extraction assembly 600 includes a container assembly 506 (that need not be hermetically sealed or air-evacuated) to draw liquid sample 180 inside container assembly 506 (as shown by arrow 634). Liquid sample 180 is moved into container assembly 506, driven by pressure differential between inside and outside container assembly 506 generated by pressure differential generator 602. Pressure differential is generated within container assembly 506 by pulling and removing air out of container assembly 506 in the direction shown by arrow 636 in a well-known manner by pressure differential generator 602. Plasma extraction assembly 600 also includes plasma extraction device 100e positioned inside container assembly 506, similar to plasma extraction assembly 500 shown and detailed in relation to FIGS. 5A to 5H.

FIG. 6C is a non-limiting exemplary exploded view illustration of container assembly 506 with adapter assembly for connection with a vacuum generator 602 in accordance with one or more embodiments of the present invention. The exploded view shown in FIG. 6C illustrates disassembled, separated components that show the cooperative working relationship, orientation, positioning, and exemplary manner of assembly of the various components of the prefabricated fixation systems in accordance with one or more embodiments of the present invention. As illustrated in FIGS. 6C to 6G, container assembly 506 is further comprised of a first detachable adapter 608 associated with a well known second detachable adapter 610 to draw fluid 180 inside container assembly 506.

First detachable adapter 608 is comprised of a fluid inlet 612 having an opening 614 that is flush with top surface 616 of detachable adapter 608, and an evacuation outlet 618 to remove air 636 from tube assembly 506 by pressure differential generator 602, with evacuation outlet 618 oriented generally perpendicular fluid inlet 612. Further included is an engagement mechanism 620 to secure detachable adapter 608 onto container assembly 506, and a filter membrane 622 to block fluid from entering into the evacuation outlet 618.

Second detachable adapter 610 is well known and is comprised of a luer lock 624 at top 626 that receives invasive probe 632, and an inlet 628 with top opening 630 to redirect fluid sample 180. Inlet 628 extends axially and is mounted onto first detachable adapter 608, with inlet 628 inserted into fluid inlet 612 of detachable adapter 608 via opening 614.

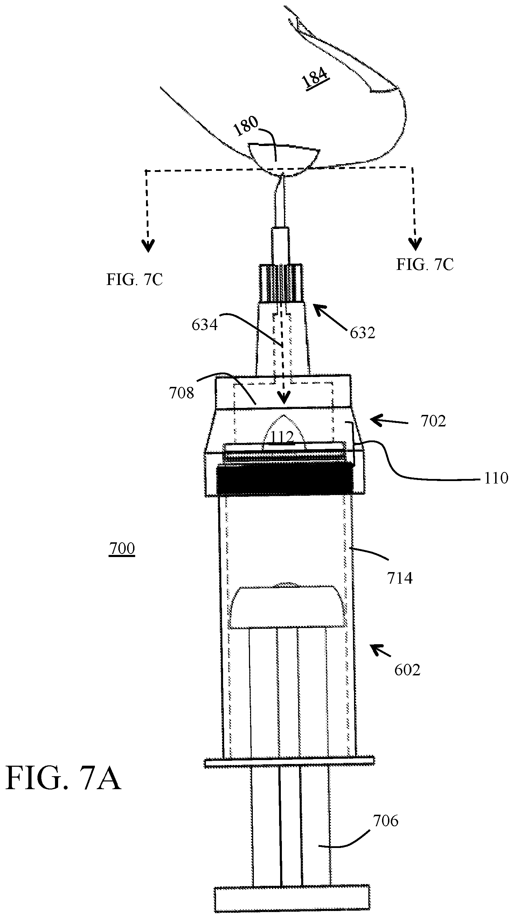

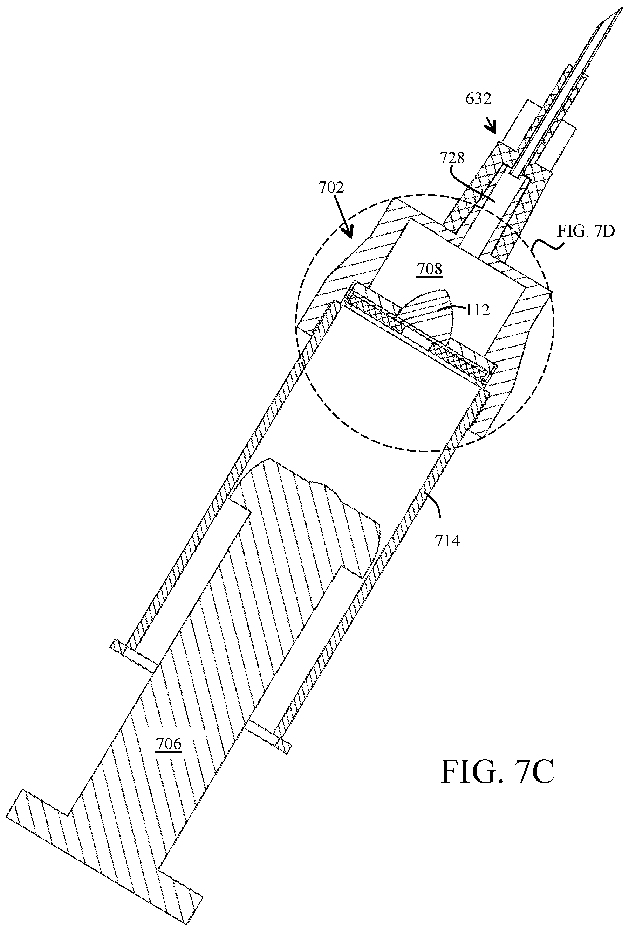

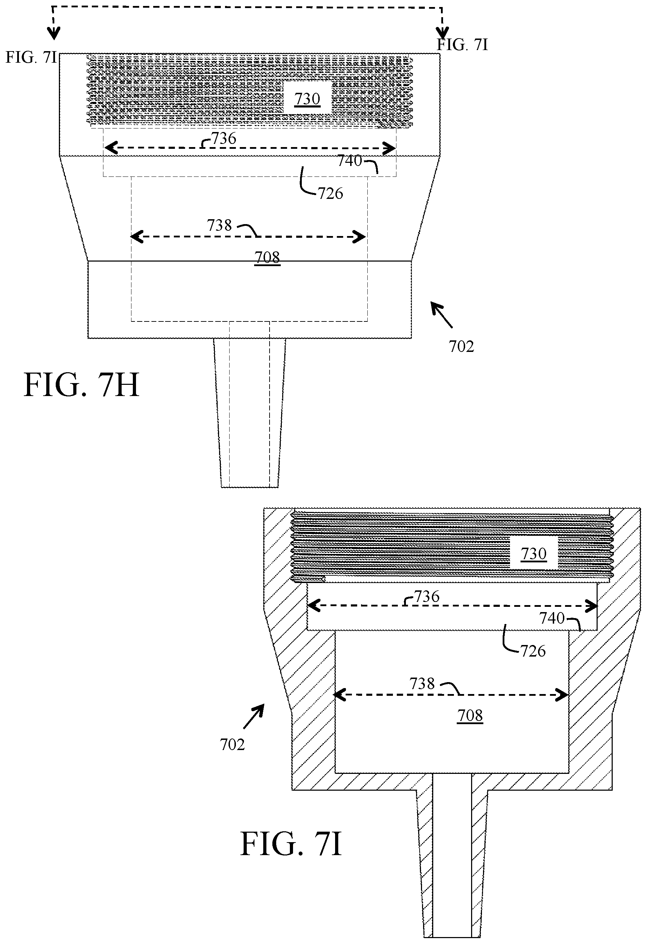

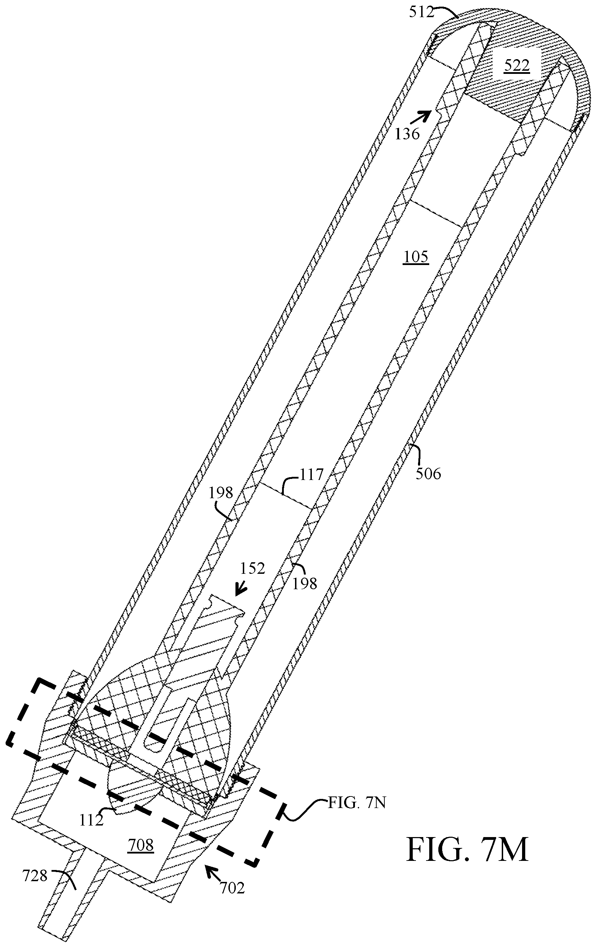

FIGS. 7A to 7P are non-limiting, exemplary illustration of various views of a plasma extraction assembly for extraction of plasma from a liquid sample in accordance with one or more embodiments of the present invention. Plasma extraction assembly 700 illustrated in FIGS. 7A to 7P includes similar corresponding or equivalent components, interconnections, functional, operational, and or cooperative relationships as plasma extraction assemblies 500 and 600 shown in FIGS. 5A to 6F, and described above. Therefore, for the sake of brevity, clarity, convenience, and to avoid duplication, the general description of FIGS. 7A to 7P will not repeat every corresponding or equivalent component, interconnections, functional, operational, and or cooperative relationships that has already been described above in relation to plasma extraction assemblies 500 and 600, and plasma extraction devices 100a, 100b, 100c, 100d, and 100e that are shown in FIGS. 1A to 6F.

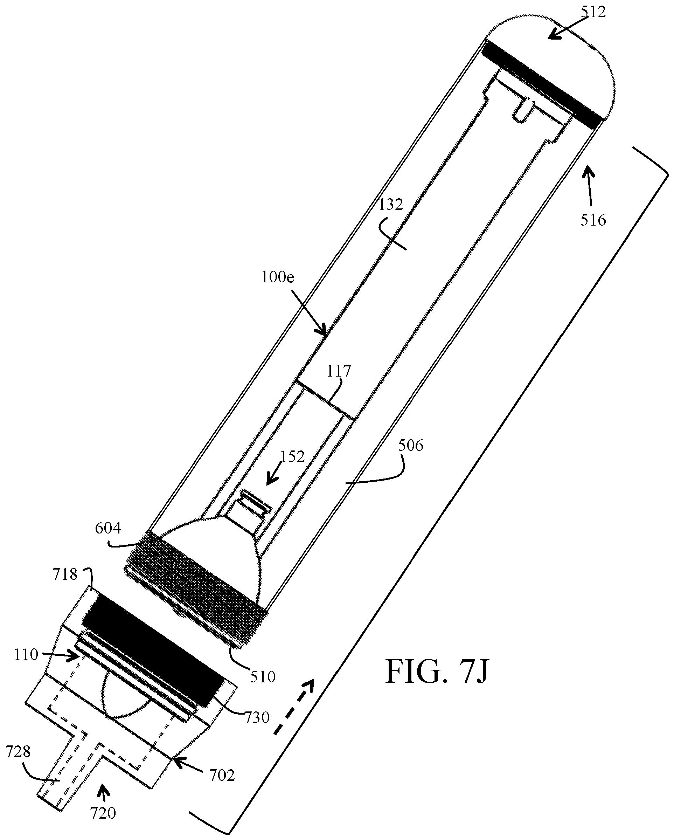

Plasma extraction assembly 700 provides a two-stage method for extraction of plasma for automated assay of the plasma loaded absorbent reservoir 116. As detailed below, first stage or phase (FIGS. 7A to 7I) uses a fluid flow facilitator in a form of a pressure differential generator 602 such as the illustrated syringe to actually obtain fluid sample 180 from a source (e.g., finger or vein) 184 and commence the process of plasma generation by the included plasma extractor module 110. The second stage or phase (FIGS. 7J to 7P) is the management of the plasma loaded extractor module 110 for automated assay of the plasma loaded absorbent reservoir 116.

As illustrated in FIGS. 7A to 7P, plasma extraction assembly 700 is comprised of pressure differential generator 602 (as the fluid flow facilitator) for moving liquid sample from a source via an invasive probe 632 and into a collection chamber 708 of an intermediate adapter 702 connected to pressure differential generator 602. A plasma extractor module 110 is positioned within intermediate adapter 702, with absorbent probe 112 of plasma extractor module 110 extended to within collection chamber 708, near egress opening 722 of invasive probe 632 for receiving liquid sample 180.

As illustrated in FIG. 7A, in this non-limiting exemplary instance, pressure differential generator 602 is a syringe. As a piston 706 of the syringe is pulled, liquid sample 180 is drawn and is moved along path indicated by arrows 634 from source 184 to within collection chamber 708 of intermediate adapter 702, with fluid sample 180 flooding over absorbent probe 112 of plasma extractor module 110. Thereafter plasma is generated as described above in relation to FIGS. 1A to 6G.

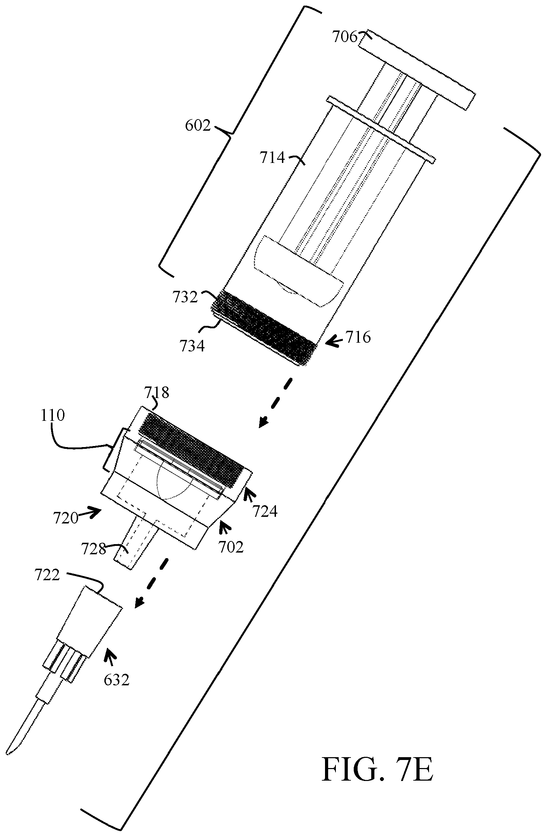

As best illustrated in FIG. 7E, once liquid sample 180 is obtained, intermediate adapter 702 is simply dethatched from pressure differential generator 602, and as illustrated in FIG. 7J, intermediate adapter 702 is then detachably fastened onto container assembly 506 that contains handler assembly 130 of plasma extraction device 100e (with o-ring 530, but obviously without a plasma extractor module 110).

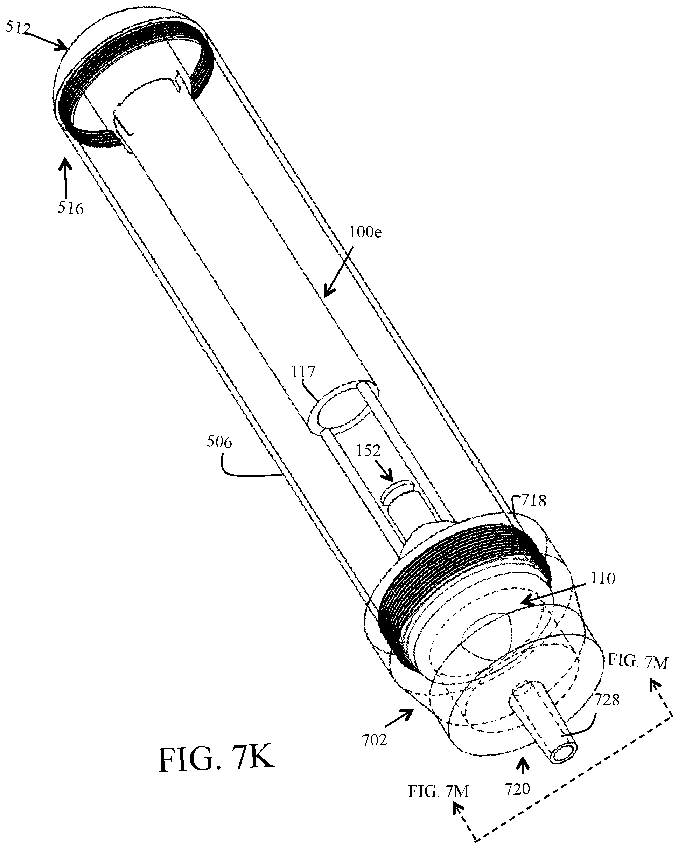

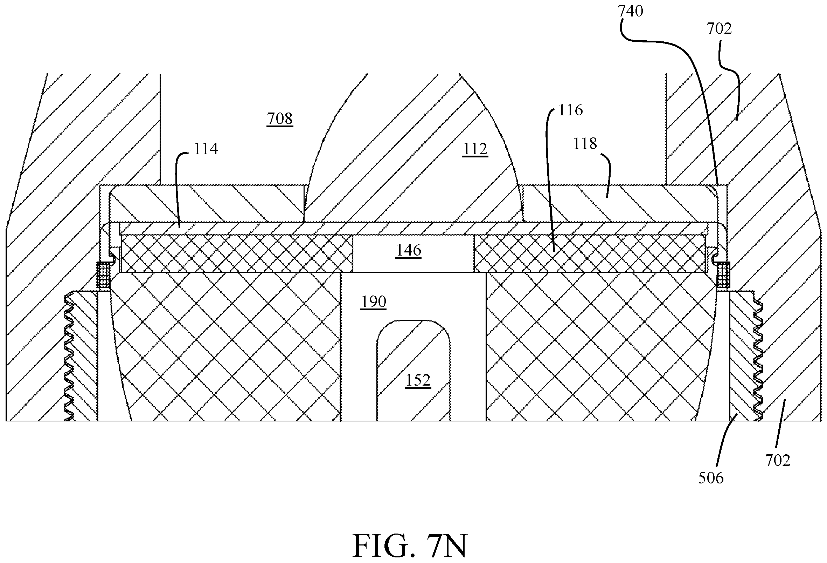

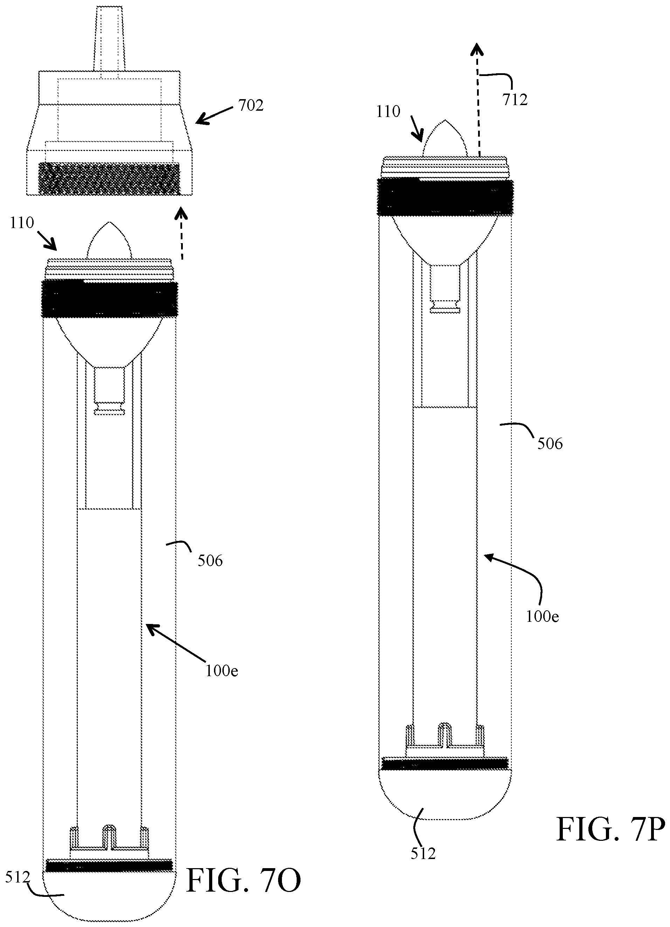

As best illustrated in FIG. 7K and 7N, as intermediate adapter 702 is transferred and fastened onto container assembly 506 and tightened, plasma extractor module 110 snaps onto a distal end 138 of handler assembly 130 as detailed in FIGS. 1A to IN, and is freed from intermediate adapter 702 as shown in FIGS. 7O. Thereafter, intermediate adapter 702 is unfastened and discarded (best shown in FIG. 7O). Thereafter, the fully assembled plasma extraction device 100e shown in FIG. 7P may be removed out of container 506 as shown by arrow 712, and used as described in relation to FIGS. 1A to 6G for assay of the plasma loaded absorbent reservoir 116 by well known automated instruments. It should be noted that container assembly 506 may be any well known container and in this non-limiting, exemplary instance, it need not have air-tight sealant closures.

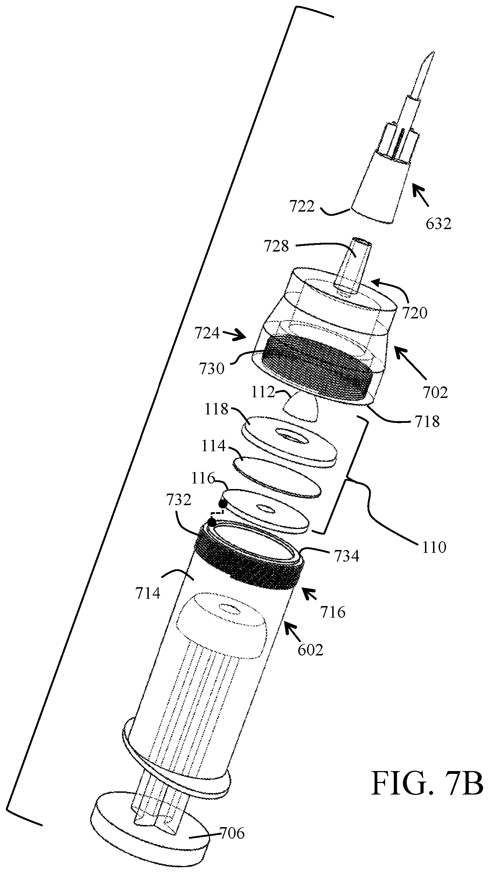

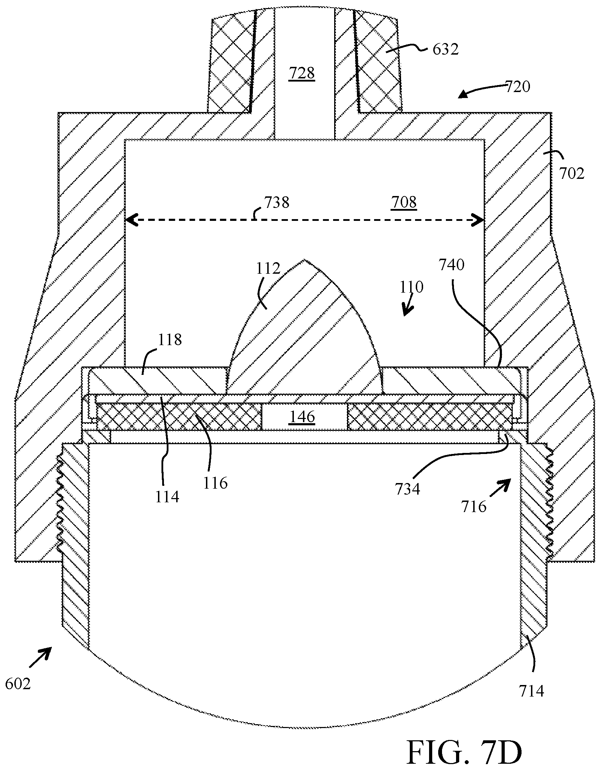

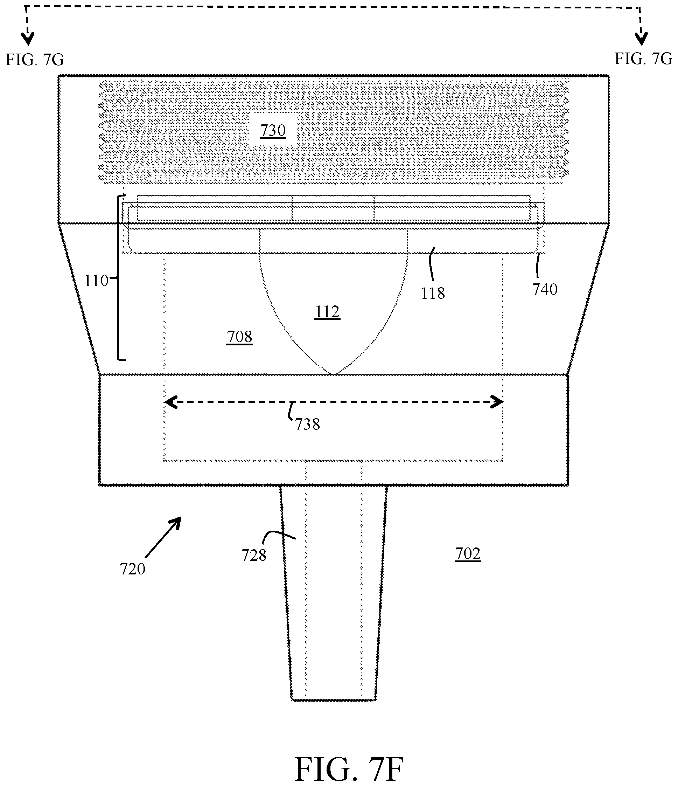

As shown in FIG. 7D, plasma extractor module 110 is secured within the intermediate adapter 702, between a top distal end 716 of a housing (or tube of the syringe) 714 of pressure differential generator 602 and a second open end 718 (FIG. 7B) of intermediate adapter 702. As shown in FIGS. 7D, and 7F to 7I, intermediate adapter 702 includes a first side 720 that receives an egress opening side 722 of invasive probe 632, and a second side 724 that includes a first compartment 726 within which plasma extractor module 110 is detachably secured, and a second compartment that is the collection chamber 708.

Intermediate adapter 702 is generally configured similar to a frustum of right circular cone, with first side 720 including a nozzle or a luer lock structure 728 extending from first side 720 for receiving egress opening side 722 of needle 632 in a well known manner (as best shown in FIG. 7B). Second side 724 of intermediate adapter 702 includes an opening 718 that receives top distal end 716 of housing 714 of pressure differential generator 602.

As shown in FIG. 7B, second side 724 includes female threads 730 that receive male threads 732 on outer circumference of housing 714. As housing 714 is fastened tightly to intermediate adapter 702, plasma extractor module 110 is pressed by periphery edge 734 of top distal end 716 of housing 714 and is tightly secured (sandwiched) within first compartment 726 of intermediate adapter 702.

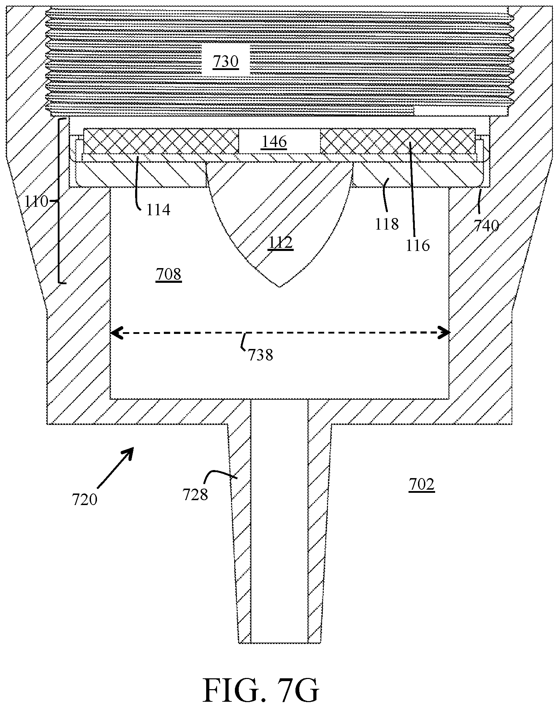

First compartment 726 of intermediate adapter 702 has a longer first diameter 736 compared to second diameter 738 second compartment 708 resulting in a distal end annular flange (or step) 740 of second compartment 708 that compress against housing 118 (FIG. 7G) of plasma extractor module 110 while absorbent reservoir 116 rests and is pressed against top distal end 716 annular flange 734 of housing 714 of pressure differential generator 602.

As in indicated above, intermediate adapter 702 is detached from pressure differential generator 602, transferred to, and detachably fastened onto container assembly 506 that houses handler assembly 130. Fastening intermediate adapter 702 to container assembly 506 detachably attaches (or reassembles) and snaps plasma extractor module 110 onto handler assembly 130, forming plasma extraction device 100e within container assembly 506. This allows intermediate adapter 702 to be detached from container assembly 506, freed from plasma extractor module 110.

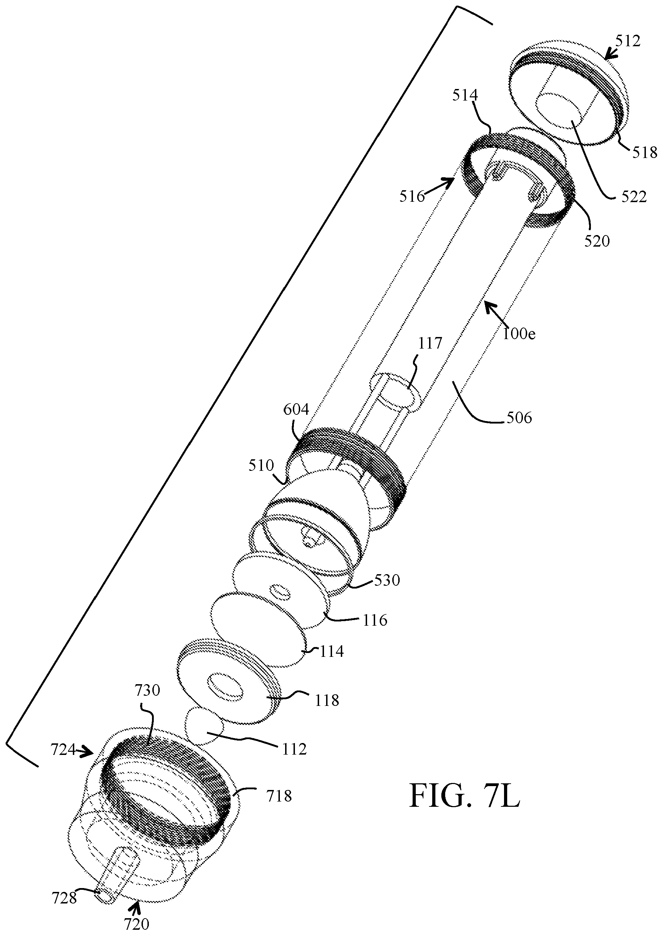

As intermediate adapter 702 is fastened tightly to container assembly 506, plasma extractor module 110 is pushed towards and tightly pressed against distal end 138 of handler assembly 130 by step 740 until plasma extractor module 110 snaps onto distal end 138 of handler assembly 130 (as detailed in FIGS. 1A to 6F), and reassembled to form plasma extraction device 100e as shown in FIGS. 1A to 6F. Accordingly, the processing of transferring intermediate adapter 702 and connecting it with container 506 is to actually reassemble plasma extraction device 100e without contacting or touching plasma extractor module 110 or any fluid contained in collector chamber 708. It should be noted that o-ring 530 (FIG. 7L) prevents any remaining fluid (if any) within collection chamber 708 from leaking into and coming in contact with the rest of handler assembly 130 as intermediate adapter 702 is connected with container assembly 506. Therefore, the value of container assembly 506 is that it facilitates easy reassembly of plasma extractor module 110 with handler assembly 130 to from plasma extraction device 100e without having to contact plasma extractor module 110 or any remaining fluids.

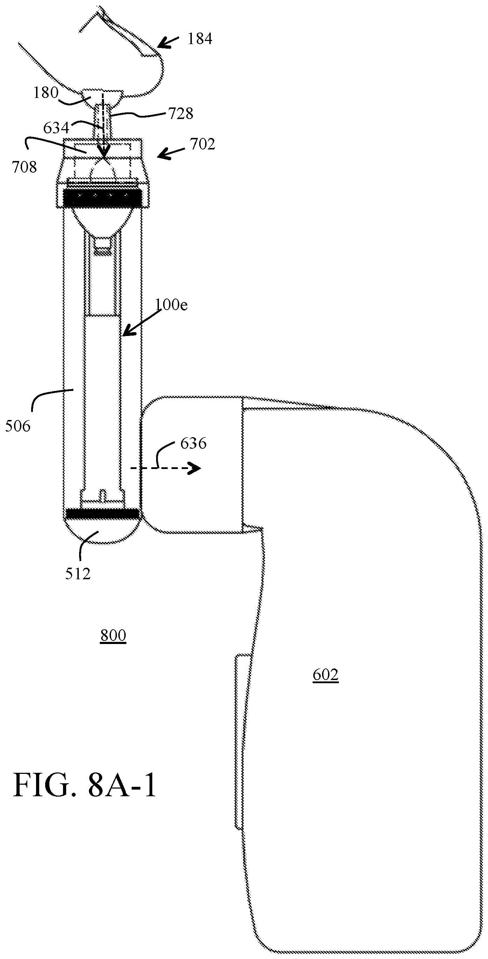

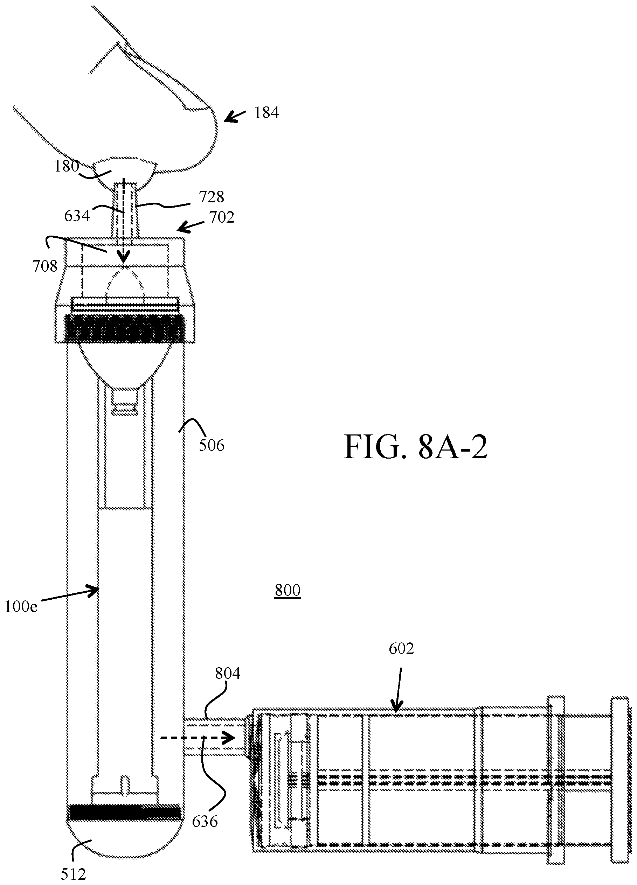

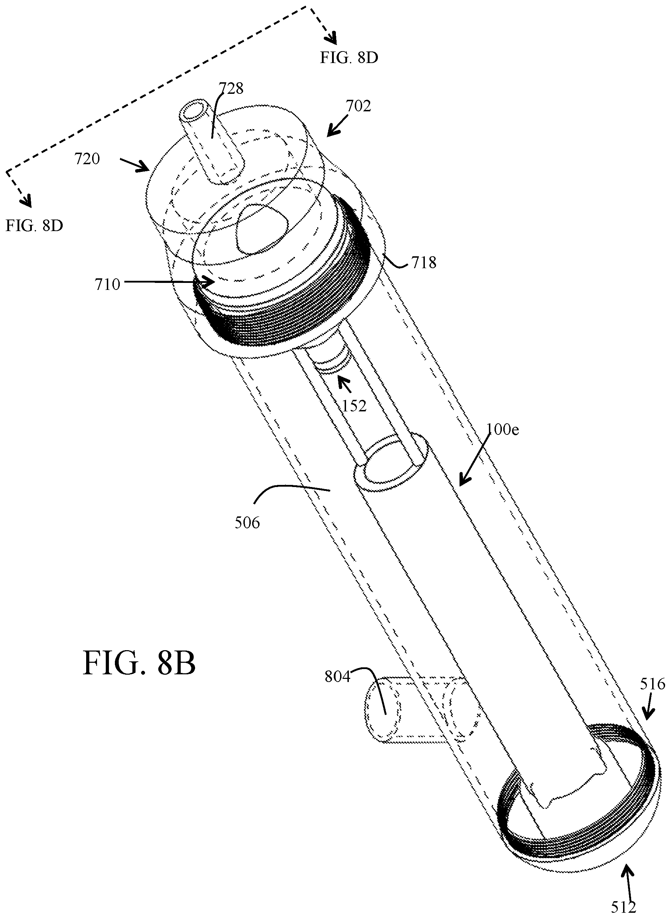

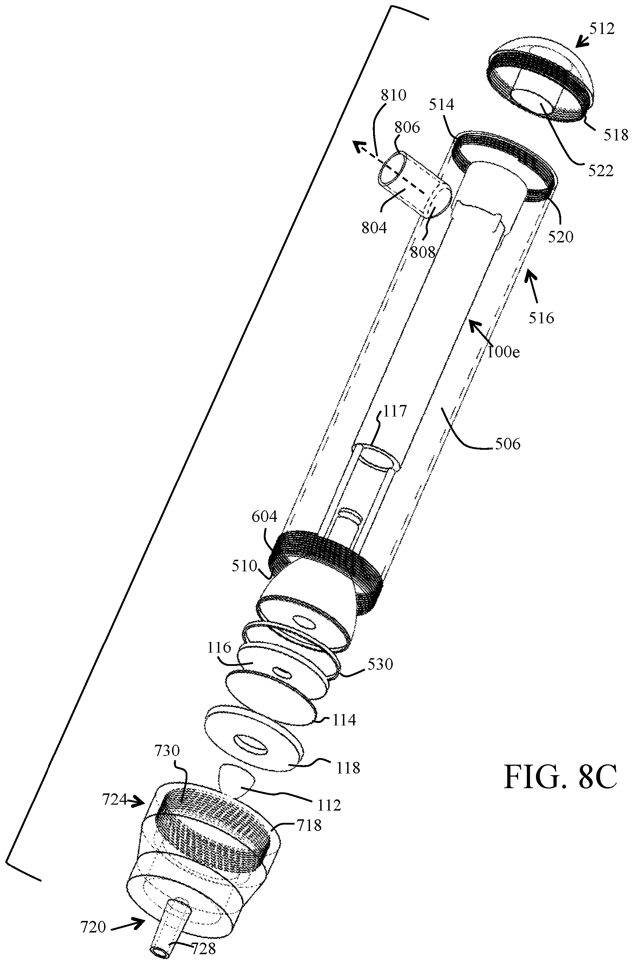

FIGS. 8A-1 to 8D are non-limiting, exemplary illustration of various views of a plasma extraction assembly for extraction of plasma from a liquid sample in accordance with one or more embodiments of the present invention. Plasma extraction assembly 800 illustrated in FIGS. 8A-1 to 8D includes similar corresponding or equivalent components, interconnections, functional, operational, and or cooperative relationships as plasma extraction assemblies 500, 600, and 700 shown in FIGS. 5A to 7P, and described above. Therefore, for the sake of brevity, clarity, convenience, and to avoid duplication, the general description of FIGS. 8A-1 to 8D will not repeat every corresponding or equivalent component, interconnections, functional, operational, and or cooperative relationships that has already been described above in relation to plasma extraction assemblies 500, 600, and 700 and plasma extraction devices 100a, 100b, 100c, 100d, and 100e that are shown in FIGS. 1A to 7P.

In this non-limiting exemplary embodiment, plasma extraction assembly 800 uses container assembly 506 with intermediate adapter 702 as top closure (shown and described in FIGS. 7J to 7N) but with the addition of a laterally extending pressure differential generation outlet 804. Accordingly, the combination of container assembly 506 and the connected pressure differential generator 602 constitute the fluid flow facilitator in this embodiment.

As illustrated in FIGS. 8A-1 to 8D, plasma extraction device 100e is positioned within container assembly 506, with container assembly 506 having intermediate adapter 702 as top closure, bottom closure 512, and a laterally extending pressure differential generation outlet 804 (near distal end 516) adapted to be detachably associated with pressure differential generator 602. This way, plasma extractor module 110 need not be separate from plasma extraction device 100e during sample collection as compared with the two stage handling and processing required by plasma extraction assembly 700 and hence, providing a much simpler handling and processing of plasma loaded absorbent reservoir 116.

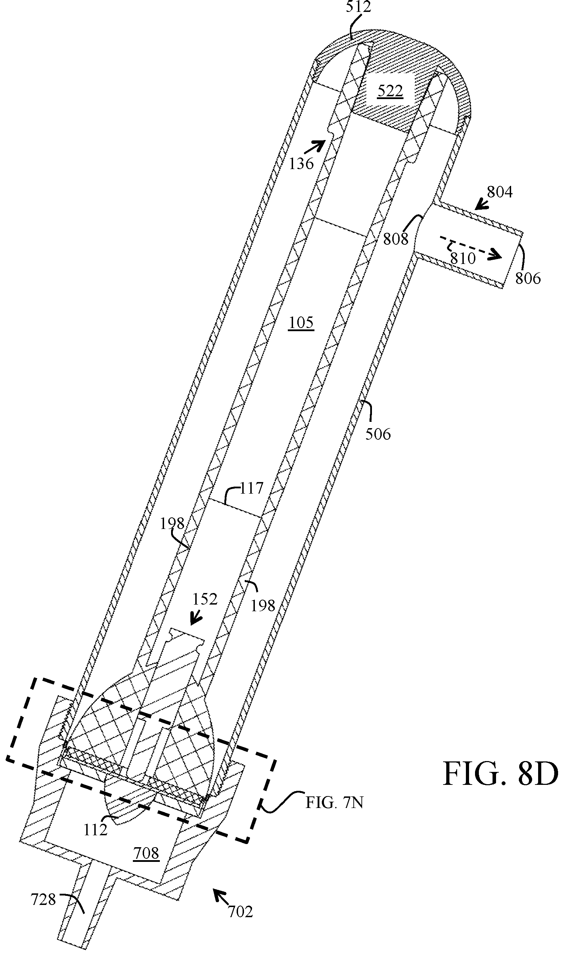

Laterally extending pressure differential generation outlet 804 is comprised of a hollow cylindrical tube structure with an external opening 806 (FIG. 8D) that opens and leads into near lower distal end 516 of container assembly 506 by an internal opening 808. Air may be evacuated from container assembly 506 in the direction shown by arrow 636 (best illustrated in FIG. 8A-1 and 8A-2) by conventional pressure differential generators connected to outlet 804.

Accordingly, after pricking a subject (e.g., a finger 184), fluid sample 180 is collected by applying pressure differential using pressure differential generator 602 as shown in FIGS. 8A-1 and 1A-2 to withdraw fluid sample 180 to within collection chamber 708 via nozzle or a luer lock structure 728 as shown by arrow 634. As the pressure within container assembly drops due to flow of air out of container assembly 506 (in the direction shown by arrow 636), fluid sample is pulled in due to the pressure differential in the direction shown by arrow 634. Once chamber 708 is flooded, pressure differential generator 602 is dethatched, top closure 702 removed, and the entire plasma extraction device 100e is removed out of container assembly 506, and handled by automated instruments as detailed above (especially in relation to FIG. 7O and 7P).

As detailed above, the same plasma extractor assembly is used with all of the above-described embodiments detailed in FIGS. 1A to 8D, which includes absorbent probe 112, separator 114, and absorbent reservoir 116. Further, the plasma extractor assembly operates the same for all embodiments shown in FIGS. 1A to 8D, enabling easy, dry transfer of fixed, predetermined quantity of plasma loaded absorbent reservoir 116 for either manual or automated assay.

Depending on the subject and the environment within which the present invention is used, the invention may be practiced using capillary action as the motive force to generate and dry-transport fixed, predetermined quantity of plasma. Alternatively, the invention may also be practiced using a combination of motive forces (e.g., actively induced pressure differential) and capillary action (an active-passive combination) to generate and dry-transport fixed, predetermined quantity of plasma. "Passive" embodiments (FIGS. 1A to 4C) are those where the motive force is not an actively induced pressure differential, but capillary action.

Further, the use of any one of the one or more embodiments disclosed in FIGS. 1A to 8D depends on the various cost factors, types of known automated instruments for assay of the plasma, the subject or patient, and the environment within which the present invention may be practiced. For example, for low cost home setting and if the subject is an adult, and if it is desired that the plasma extraction device be auto handled by automated instruments for assay, "passive" embodiment with capillary action as the motive force detailed in embodiments in relation to FIGS. 1A to 2R may be used. In those instances, a patient may simply use plasma extraction devices 100a or 100b, position either back into tube 102 and send to lab for automated processing and assay of plasma loaded (but dry) absorbent reservoir 116. As another example, if small, compact form-factor is important (e.g., for an emergency kit used in remote locations) where automated handling of the plasma loaded absorbent reservoir 116 for assay is a secondary factor, then embodiments disclosed in FIGS. 3A to 4C may be used instead, and sent to a lab for assay of plasma loaded and dry absorbent reservoir 116. Of course, the use of plasma extraction devices with fluid flow facilitator ("active" motive force) in any setting such as home, clinical, or emergency would be best as plasma extraction assemblies improve patient comfort, reduced pain, and improve overall patient experience.

The specification is not confined to the disclosed embodiments. Therefore, while exemplary illustrative embodiments of the invention have been described, numerous variations and alternative embodiments will occur to those skilled in the art. For example, the number, sizes, and shapes of the absorbent probe, separator, and absorbent reservoir may be varied to optimize the extraction process of plasma (e.g., quantity extracted and stored, plasma purity, etc.), which may dependent on the type of fluid being handled. For example, assuming blood is the fluid sample and it contains 50% red/white blood cells, the maximum amount of plasma extracted would be 50% (assuming ideal conditions) and hence, the number, sizes, and shapes of the absorbent probe, separator, and absorbent reservoir used may be varied to optimize plasma extraction. As another example, the forces that are using to move fluid near probe 112 are shown to be vacuum, or positive pressure from the blood stream of a patient, but could also include positive pressure or vacuum from any source (liquid or gas). Vacuum is also illustrated as a mechanism to assist in pulling fluid through the separator, but other assisting forces could also be used including compression of the probe and separator to help "wring-out" the plasma fluid.

It should further be noted that throughout the entire disclosure, the labels such as left, right, front, back, top, inside, outside, bottom, forward, reverse, clockwise, counter clockwise, up, down, or other similar terms such as upper, lower, aft, fore, vertical, horizontal, oblique, proximal, distal, parallel, perpendicular, transverse, longitudinal, etc. have been used for convenience purposes only and are not intended to imply any particular fixed direction, orientation, or position. Instead, they are used to reflect relative locations/positions and/or directions/orientations between various portions of an object.

In addition, reference to "first," "second," "third," and etc. members throughout the disclosure (and in particular, claims) is not used to show a serial or numerical limitation but instead is used to distinguish or identify the various members of the group.

## Claims

1. A device (100a) for extraction of plasma from a liquid sample, comprising:
a first absorbent member (112) that wicks the liquid sample;
a second absorbent member (116) that retains a fixed, predetermined quantity of plasma;
a separator (114) placed in physical contact between the first absorbent member (112) and the second absorbent member (116) for extracting plasma from the liquid sample;
a first side (140) of the separator (114) is comprised of a first membrane and a second side (144) of the separator is comprised of a second membrane;
the first membrane is comprised of a lower porosity construction with enhanced hydrophilic properties relative to the first absorbent member, facilitating capillary action from the first absorbent member to the first membrane;
the second membrane is comprised of a higher porosity construction and lesser hydrophilic properties relative to the second absorbent member, facilitating capillary action from the second membrane to the second absorbent member;
wherein: the plasma loaded second absorbent member (116) is dry-transferred for assay.

2. The device for extraction of plasma from a liquid sample as set forth in claim 1, wherein:
the first absorbent member wicks the liquid sample by a first capillary action from a liquid source.

3. The device for extraction of plasma from a liquid sample as set forth in claim 1, wherein:
the first absorbent member (112) has a volume of 10 to 500 µl with a porosity construction of about 40 microns which is greater than the volume and porosity construction of the separator (114).

4. The device for extraction of plasma from a liquid sample as set forth in claim 1, wherein:
the first absorbent member (112) has a side pressed against the first side (140) of the separator (114), enabling transfer of the liquid sample by a capillary action from the first absorbent member (112) to the first side (140) of the separator (114).

5. The device for extraction of plasma from a liquid sample as set forth in claim 1, wherein:
the separator (114) separates plasma of the transferred liquid sample, moved from the first side (140) of the separator to the second side (140) of the separator (114).

6. The device for extraction of plasma from a liquid sample as set forth in claim 1, wherein:
the second absorbent member (116) has a side pressed against the second side (144) of the separator for wicking the plasma from the second side (144) of the separator by a capillary action.

7. The device for extraction of plasma from a liquid sample as set forth in claim 1, wherein:
the first absorbent member (112) wicks the liquid sample from a liquid source by a first capillary action;
the separator (114) wicks the liquid sample from the first absorbent member (112) by a second capillary action driven by differences in porosity construct between the first side (140) of the separator and the first absorbent member (112), and extracts plasma; and
the second absorbent member (144) wicks plasma from the separator (114) by a third capillary action due to differences between hydrophilic properties of the second absorbent member (116) and the second side (144) of the separator.

8. The device for extraction of blood plasma as set forth in claim 1, wherein:
the first (112) and the second (116) absorbent members are hydrophilic and have high porosity constructions.

9. The device for extraction of blood plasma as set forth in claim 1, wherein:
the second absorbent member (116) extracts a specific quantity of stored plasma from within the second membrane due to a fixed size of the absorbent member.

## Patentansprüche

1. Vorrichtung (100a) zum Extrahieren von Plasma aus einer flüssigen Probe, umfassend:
ein erstes Absorptionselement (112), das die flüssige Probe aufnimmt;
ein zweites Absorptionselement (116), das eine feste vorbestimmte Menge von Plasma zurückhält;
einen Separator (114), der in körperlichem Kontakt zwischen dem ersten Absorptionselement (112) und dem zweiten Absorptionselement (116) angeordnet ist, um aus der flüssigen Probe Plasma zu extrahieren;
wobei eine erste Seite (140) des Separators (114) aus einer ersten Membran aufgebaut ist und eine zweite Seite (144) des Separators aus einer zweiten Membran aufgebaut ist;
die erste Membran aus einer niedrig-porösen Konstruktion mit verbesserten hydrophilen Eigenschaften relativ zu dem ersten Absorptionselement aufgebaut ist, was eine Kapillarwirkung von dem ersten Absorptionselement zu der ersten Membran erleichtert;
die zweite Membran aus einer höher-porösen Konstruktion und mit geringeren hydrophilen Eigenschaften relativ zu dem zweiten Absorptionselement aufgebaut ist, was eine Kapillarwirkung von der zweiten Membran zu dem zweiten Absorptionselement erleichtert;
wobei das auf das zweite Absorptionselement (116) geladene Plasma zur Prüfung trocken-transferiert wird.

2. Die Vorrichtung zum Extrahieren von Plasma aus einer flüssigen Probe nach Anspruch 1, wobei:
das erste Absorptionselement die flüssige Probe durch eine erste Kapillarwirkung von einer Flüssigkeitsquelle aufnimmt.

3. Die Vorrichtung zum Extrahieren von Plasma aus einer flüssigen Probe nach Anspruch 1, wobei:
das erste Absorptionselement (112) ein Volumen von 10 bis 500µl mit einer Porositätskonstruktion von etwa 40 Mikrometer hat, die größer sind als das Volumen und die Porositätskonstruktion des Separators (114).

4. Die Vorrichtung zum Extrahieren von Plasma aus einer flüssigen Probe nach Anspruch 1, wobei:
das erste Absorptionselement (112) eine gegen die erste Seite (140) des Separators (114) gepresste Seite hat, was die Übertragung der flüssigen Probe durch Kapillarwirkung von dem ersten Absorptionselement (112) zu der ersten Seite (140) des Separators (114) ermöglicht.

5. Die Vorrichtung zum Extrahieren von Plasma aus einer flüssigen Probe nach Anspruch 1, wobei:
der Separator (114) Plasma der überführten flüssigen Probe, das er von der ersten Seite (140) des Separators zu der zweiten Seite (140) des Separators (114) bewegt hat, abtrennt.

6. Die Vorrichtung zum Extrahieren von Plasma aus einer flüssigen Probe nach Anspruch 1, wobei:
das zweite Absorptionselement (116) eine gegen die zweite Seite (144) des Separators gepresste Seite hat, um das Plasma von der zweiten Seite (144) des Separators durch Kapillarwirkung aufzunehmen.

7. Die Vorrichtung zum Extrahieren von Plasma aus einer flüssigen Probe nach Anspruch 1, wobei:
das erste Absorptionselement (112) die flüssige Probe von einer Flüssigkeitsquelle durch eine erste Kapillarwirkung aufnimmt;
der Separator (114) die flüssige Probe von dem ersten Absorptionselement (112) durch eine zweite Kapillarwirkung aufnimmt, die durch Differenzen in der Porositätskonstruktion zwischen der ersten Seite (140) des Separators und dem ersten Absorptionselement (112) betrieben wird, und Plasma extrahiert; und
das zweite Absorptionselement (114) Plasma von dem Separator (114) durch eine dritte Kapillarwirkung aufgrund Differenzen zwischen hydrophilen Eigenschaften des zweiten Absorptionselements (116) und der zweiten Seite (144) des Separators aufnimmt.

8. Die Vorrichtung zum Extrahieren von Blutplasma nach Anspruch 1, wobei:
die ersten (112) und zweiten (116) Absorptionselemente hydrophil sind und hochporöse Konstruktionen haben.

9. Die Vorrichtung zum Extrahieren von Blutplasma nach Anspruch 1, wobei:
das zweite Absorptionselement (116) eine spezifische Menge des gespeicherten Plasmas aus innerhalb der zweiten Membran aufgrund einer festen Größe des Absorptionselements extrahiert.

## Revendications

1. Dispositif (100a) d'extraction de plasma à partir d'un échantillon liquide, comprenant :
un premier élément absorbant (112) qui draine l'échantillon de liquide ;
un second élément absorbant (116) qui retient une quantité fixe et prédéterminée de plasma ;
un séparateur (114) placé en contact physique entre le premier élément absorbant (112) et le second élément absorbant (116) pour extraire du plasma à partir de l'échantillon de liquide ;
un premier côté (140) du séparateur (114) est composé d'une première membrane et un second côté (144) du séparateur est composé d'une deuxième membrane ;
la première membrane est constituée d'une structure de porosité inférieure avec des propriétés hydrophiles améliorées par rapport au premier élément absorbant, facilitant une action capillaire du premier élément absorbant à la première membrane ;
la seconde membrane est constituée d'une structure de porosité supérieure et de propriétés hydrophiles moindres par rapport au second élément absorbant, facilitant une action capillaire de la seconde membrane vers le second élément absorbant ;
dans lequel : le second élément absorbant chargé de plasma (116) est transféré à sec pour un dosage.

2. Dispositif d'extraction de plasma à partir d'un échantillon de liquide selon la revendication 1, dans lequel :
le premier élément absorbant draine l'échantillon de liquide par une première action capillaire à partir d'une source de liquide.

3. Dispositif d'extraction de plasma à partir d'un échantillon de liquide selon la revendication 1, dans lequel :
le premier élément absorbant (112) a un volume de 10 à 500 µl avec une construction de porosité d'environ 40 microns qui est plus grande que la construction de volume et de porosité du séparateur (114).

4. Dispositif d'extraction de plasma à partir d'un échantillon de liquide selon la revendication 1, dans lequel :
le premier élément absorbant (112) a un côté pressé contre le premier côté (140) du séparateur (114), en permettant un transfert de l'échantillon de liquide par une action capillaire du premier élément absorbant (112) vers le premier côté (140) du séparateur (114).

5. Dispositif d'extraction de plasma à partir d'un échantillon de liquide selon la revendication 1, dans lequel :
le séparateur (114) sépare le plasma de l'échantillon de liquide transféré, déplacé depuis le premier côté (140) du séparateur vers le second côté (140) du séparateur (114).

6. Dispositif d'extraction de plasma à partir d'un échantillon de liquide selon la revendication 1, dans lequel :
le second élément absorbant (116) a un côté pressé contre le second côté (144) du séparateur pour drainer le plasma à partir du second côté (144) du séparateur par une action capillaire.

7. Dispositif d'extraction de plasma à partir d'un échantillon de liquide selon la revendication 1, dans lequel :
le premier élément absorbant (112) draine l'échantillon de liquide à partir d'une source de liquide par une première action capillaire ;
le séparateur (114) draine l'échantillon de liquide à partir du premier élément absorbant (112) par une deuxième action capillaire entraînée par des différences de construction de porosité entre le premier côté (140) du séparateur et le premier élément absorbant (112), et extrait du plasma ; et
le second élément absorbant (144) draine du plasma à partir du séparateur (114) par une troisième action capillaire en raison de différences entre des propriétés hydrophiles du second élément absorbant (116) et du second côté (144) du séparateur.

8. Dispositif d'extraction de plasma sanguin selon la revendication 1, dans lequel :
les premier (112) et second (116) éléments absorbants sont hydrophiles et ont des constructions de porosité élevée.

9. Dispositif d'extraction de plasma sanguin selon la revendication 1, dans lequel :
le second élément absorbant (116) extrait une quantité spécifique de plasma stocké depuis l'intérieur de la seconde membrane en raison d'une taille fixe de l'élément absorbant.
